(19) Europäisches Patentamt
European Patent Office
Office européen des brevets

(11) EP 2 207 789 B1

(12) # EUROPEAN PATENT SPECIFICATION

(45) Date of publication and mention of the grant of the patent:
**11.06.2014 Bulletin 2014/24**

(21) Application number: **08831106.3**

(22) Date of filing: **12.09.2008**

(51) Int Cl.:
***G01N 33/543*** *(2006.01)* ***G01N 33/58*** *(2006.01)*
***B82Y 15/00*** *(2011.01)*

(86) International application number:
**PCT/US2008/010651**

(87) International publication number:
**WO 2009/035657 (19.03.2009 Gazette 2009/12)**

(54) **FUNCTIONALIZED NANOPARTICLES AND METHOD**

FUNKTIONALISIERTE NANOPARTIKEL UND VERFAHREN

NANOPARTICULES FONCTIONNALISÉES ET PROCÉDÉ CORRESPONDANT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority:
**12.09.2007 US 971887 P**
**12.09.2007 US 971885 P**
**19.09.2007 US 973644 P**
**03.12.2007 PCT/US2007/024750**
**05.12.2007 US 992598 P**
**21.12.2007 US 16227 P**
**25.06.2008 PCT/US2008/007902**
**28.07.2008 US 83998 P**

(43) Date of publication of application:
**21.07.2010 Bulletin 2010/29**

(60) Divisional application:
**13004764.0**

(73) Proprietor: **QD Vision, Inc.**
**Lexington, MA 02421 (US)**

(72) Inventors:
- **BREEN, Craig**
  **Somerville, MA (US)**
- **COX, Marshall**
  **North Haven, CT 06473 (US)**
- **STECKEL, Jonathan, S.**
  **Bedford**
  **MA 01730 (US)**

(74) Representative: **Grünecker, Kinkeldey, Stockmair & Schwanhäusser**
**Leopoldstrasse 4**
**80802 München (DE)**

(56) References cited:
**US-A1- 2003 059 635**
**US-A1- 2004 023 261**
**US-A1- 2004 048 241**
**US-A1- 2004 265 571**
**US-A1- 2005 266 246**
**US-A1- 2005 266 246**
**US-A1- 2006 216 759**

- **DOUSSINEAU T ET AL: "Synthesis of phosphonic acids with the semicarbazide group for the functionalization of metal oxide mid zeolite nanoparticles", SYNLETT 20040809 DE, no. 10, 9 August 2004 (2004-08-09), pages 1735-1738, XP002615460, ISSN: 0936-5214**
- **DIAMENTE P R ET AL: "Bioconjugation of Ln<3+>-Doped LaF3 nanoparticles to avidin", LANGMUIR 20060214 AMERICAN CHEMICAL SOCIETY US, vol. 22, no. 4, 14 February 2006 (2006-02-14), pages 1782-1788, XP002615461, DOI: DOI:10.1021/LA052589R**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 207 789 B1

## Description

### TECHNICAL FIELD

The present invention relates to the technical field of nanoparticles and more particularly nanoparticles including ligands and related methods.

### BACKGROUND

A predominant method for the synthesis of colloidal quantum dots involves reactions done in high boiling solvents, such as trioctylphosphine oxide (TOPO), trioctylphosphine (TOP), aliphatic phosphonic or carboxylic acids, and aliphatic amine species. The ligand capping groups on the surface of the quantum dots are, therefore, believed to be a statistical distribution of TOPO, TOP, acid, and amine. Throughout the quantum dot literature, in order to affect surface chemistry changes on a particular quantum dot sample (e.g. making water-soluble quantum dots), typical procedures involve cap exchange reactions, whereby already synthesized quantum dots (core or core-shell) are placed in a solution of another ligand and heated for an extended period of time in order to drive off the existing ligands and replace them with the alternate species. These procedures can be detrimental to maintaining the optical properties of the quantum dots and often result in drastically reduced emission effciencies and stability.

Alternative techniques utilize self-assembled micelles that surround and inter-digitate with the native quantum dot surface ligands. The drawback of these methods include the requirement for polar solvent environments to generate the encapsulating micelle, and thus limiting the technique to aqueous based applications, such as biological tagging and imaging.

Thus, there remains a need for a semiconductor nanocrystal including functionalized ligands that are compatible with an organic based solvent system, and methods for preparing same.

US 2006/0216759 is directed to semiconductor nanocrystals coated with a coating comprising a ligand.

US 2003/0059635 relates to fluorescent nanocrystals coated with an imidazole-containing compound.

US 2005/0266246 describes nanocrystals comprising an inorganic core and an organic coating layer.

Doussineau et al., Synlett 2004, 10, pages 1735 to 1738 describe the functionalization of metal oxides and zeolite nanoparticles with phosphonic acids.

Diamente et al., Langmuir 2006, 22, pages 1782 to 1788 describe lanthanide-doped $LaF_3$ nanoparticles.

### SUMMARY OF THE INVENTION

The present invention relates to nanoparticles including one or more ligands attached to a surface of the nanoparticle. The present invention also relates to methods for preparing a nanoparticle in the presence of one or more ligands. The present invention is based on claims 1 to 25.

In accordance with one aspect of the present invention, there is provided a nanoparticle according to claim 1 including one or more chemically distinct native ligands attached to a surface thereof, at least one of said ligands being represented by the formula:

$$X\text{-}Sp\text{-}Z$$

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle, and/or (ii) a group that is cyclic, halogenated, and/or polar a-protic, wherein Z in all cases is not reactive upon exposure to light.

As used herein, "native ligand" refers to a ligand that attaches or coordinates to a nanoparticle surface during the growth or overcoating thereof with an over coating material comprising a semiconductor material. Ligands are considered chemically distinct when they have different chemical compositions.

In certain embodiments, Z does not render the nanoparticle dispersible in a liquid medium that includes water.

In certain embodiments, a reactive group can comprise a functional, bifunctional, or polyfunctional reagent, and/or a reactive chemical group.

As used herein, "reactive chemical group" refers to a chemical group that can react with one or more other groups or species. Examples of reactive chemical groups include functional substituent groups. Examples of functional substituent groups include, but are not limited to, thiol, carboxyl, hydroxyl, amino, amine, sulfo, bifunctional groups, polyfunctional groups, etc.)

In certain embodiments, a cyclic group can comprise a saturated or unsaturated cyclic (including, a single ring, a bicyclic structure, a multi-cyclic structure) compound or aromatic compound. In certain embodiments, the cyclic group can include at least one hetero-atom. In certain embodiments, the cyclic group can include at least one substituent group (including, for example a reactive chemical group, an organic group (alky, aryl). Other examples of cyclic groups are provided herein.

In certain embodiments, a halogenated group can comprise a fluorinated group, a perfluorinated group, a chlorinated group, a perchlorinated group, a brominated group, a perbrominated group, an iodinated group, a periodinated group. Other examples of halogenated groups are provided herein.

In certain embodiments, a polar a-protic group can comprise a ketone, aldehyde, amide, urea, urethane, or an imine. Other examples of polar a-protic groups are provided herein.

In certain embodiments, a nanoparticle can comprise a semiconductor material.

In certain embodiments, a nanoparticle can comprise a core comprising a first material and a shell (or coating material) disposed over at least a portion of a surface of the core, the shell comprising a second material. In certain embodiments, the first material comprises a semiconductor material. In certain embodiments, the second material comprises a semiconductor material. In certain embodiments, one or more additional shells are disposed over at least a portion of a surface of the shell.

In certain preferred embodiments, a nanoparticle comprises a semiconductor nanocrystal. (A semiconductor nanocrystal is also referred to herein as a quantum dot.) In certain embodiments, the semiconductor nanocrystal can comprise a core comprising a first material and a shell (or coating material) disposed over at least a portion of a surface of the core, the shell comprising a second material. Preferably, the second material comprises a nanocrystalline semiconductor material. In certain embodiments, one or more additional shells are disposed over at least a portion of a surface of the shell. Additional discussion of nanoparticles and semiconductor nanocrystals is provided elsewhere herein.

Preferred ligands comprise benzylphosphonic acid, benzylphosphonic acid including at least one substituent group on the ring of the benzyl group, a conjugate base of such acids, and mixtures including one or more of the foregoing. In certain embodiments, a ligand comprises 4-hydroxybenzylphosphonic acid, a conjugate base of the acid, or a mixture of the foregoing. In certain embodiments, a ligand comprises 3, 5-di-*tert*-butyl-4-hydroxybenzylphosphonic acid, a conjugate base of the acid, or a mixture of the foregoing.

Other preferred ligands include ligands being represented by the formula X-Sp-Z comprising an organic amine including a terminal hydroxyl group or a fluorinated organic amine.

In certain preferred embodiments, a nanoparticle comprises a semiconductor nanocrystal core comprising a first semiconductor material having an overcoating material comprising a second semiconductor material disposed on at least a portion of a surface of the core, wherein the overcoating material is grown thereon in the presence of one or more of the ligands described herein.

In certain embodiments, a nanoparticle can include two or more chemically distinct native ligands attached to a surface thereof, at least one of said ligands being represented by the formula:

X-Sp-Z,

wherein X, Sp, and Z are as described herein.

In certain embodiments, a nanoparticle can include two or more chemically distinct ligands attached to a surface thereof, a first ligand is represented by the formula:

N-Sp-Z

wherein N represents a primary amine group, a secondary amine group, an amide group; and
a second ligand is represented by the formula:

Y-Sp-Z

wherein Y represents a phosphonic or arsonic acid group, a phosphinic or arsinic acid group, an arsenate group, a phosphine or arsine oxide group; and
wherein Sp, and Z are as described herein, and wherein each of Sp and Z on the first ligand and on the second ligand can be the same or different.

In certain embodiments, Z does not render the nanoparticle dispersible in a liquid medium that includes water.

The nanoparticle can be as described above and elsewhere herein.

Preferred ligands include benzylphosphonic acid, benzylphosphonic acid including at least one substituent group on the ring of the benzyl group, a conjugate base of such acids, and mixtures including one or more of the foregoing. In certain embodiments, a ligand comprises 4-hydroxybenzylphosphonic acid, a conjugate base of the acid, and a mixture

of the foregoing. In certain embodiments, a ligand comprises 3, 5-di-*tert*-butyl-4-hydroxybenzylphosphonic acid, a conjugate base of the acid, or a mixture of the foregoing.

Other preferred ligands include ligands being represented by the formula X-Sp-Z comprising an organic amine including a terminal hydroxyl group or a fluorinated organic amine.

In accordance with another aspect of the present invention, there is provided a method for functionalizing a nanoparticle according to claim 2. The method comprises reacting precursors for forming a nanoparticle having a predetermined composition in the presence of one or more chemically distinct ligands, at least one of the ligands being represented by the formula:

X-Sp-Z

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle and/or (ii) a group that is cyclic, halogenated, and/or polar a-protic, wherein Z in all cases is not reactive upon exposure to light.

In certain embodiments, Z does not render the nanoparticle dispersible in a liquid medium that includes water.

In certain embodiments, a reactive group can comprise a functional, bifunctional, or polyfunctional reagent, and/or a reactive chemical group.

In certain embodiments, a cyclic group can comprise a saturated or unsaturated cyclic (including a single ring, a bicyclic structure, a multi-cyclic structure) compound or aromatic compound. In certain embodiments, the cyclic group can include at least one hetero-atom. In certain embodiments, the cyclic group can include at least one substituent group (including, for example, a reactive chemical group, an organic group (alky, aryl) Other examples of cyclic groups are provided herein.

In certain embodiments, a halogenated group can comprise a fluorinated group, perfluorinated group, a chlorinated group, a perchlorinated group, a brominated group, a perbrominated group, an iodinated group, a periodinated group, etc. Other examples of halogenated groups are provided herein.

In certain embodiments, a polar a-protic group can comprise a ketone, aldehyde, amide, urea, urethane, or an imine. Other examples of polar a-protic groups are provided herein.

In certain embodiments, the predetermined composition of the nanoparticle comprises a semiconductor material.

In certain preferred embodiments, the predetermined composition comprises one or more metals and one or more chalcogens or pnictogens

In certain embodiments, precursors include one or more metal-containing precursors and one or more chalcogen-containing or pnictogen-containing precursors.

In certain embodiments, a nanoparticle can comprise a core comprising a first material and a shell (or coating material) disposed over at least a portion of a surface of the core, the shell comprising a second material. In certain embodiments, the first material comprises a semiconductor material. In certain embodiments, the second material comprises a semiconductor material. In certain embodiments, one or more additional shells are disposed over at least a portion of a surface of the shell.

In certain preferred embodiments, a nanoparticle comprises a semiconductor nanocrystai. In certain embodiments, the semiconductor nanocrystal can comprise a core comprising a first material and a shell (or coating material) disposed over at least a portion of a surface of the core, the shell comprising a second material. Preferably, the second material comprises a nanocrystalline semiconductor material. In certain embodiments, one or more additional shells are disposed over at least a portion of a surface of the shell.

Preferred ligands include benzylphosphonic acid, benzylphosphonic acid including at least one substituent group on the ring of the benzyl group, a conjugate base of such acids, and mixtures including one or more of the foregoing. In certain embodiments, a ligand comprises 4-hydroxybenzylphosphonic acid, a conjugate base of the acid, and mixtures including one or more of the foregoing. In certain embodiments, a ligand comprises 3, 5-di-*tert*-butyl-4-hydroxybenzylphosphonic acid, a conjugate base of the acid, or a mixture of the foregoing.

Other preferred ligands include ligands being represented by the formula X-Sp-Z comprising an organic amine including a terminal hydroxyl group or a fluorinated organic amine.

In certain embodiments, the precursors are reacted in the presence of two or more chemically distinct ligands, at least one of said ligands being represented by the formula:

X-Sp-Z,

wherein X, Sp, and Z are as described herein.

In certain embodiments, the precursors comprise one or more metal-containing precursors and one or more chalcogen-containing precursors or pnictogen-containing precursors.

In certain embodiments, the precursors are reacted in the presence of two or more chemically distinct ligands, wherein a first ligand is represented by the formula:

N-Sp-Z

wherein N represents a primary amine group, a secondary amine group, an amide group; and a second ligand is represented by the formula:

Y-Sp-Z

wherein Y represents a phosphonic or arsonic acid group, a phosphinic or arsinic acid group, an arsenate group, a phosphine or arsine oxide group; and wherein Sp, and Z are as described herein, and wherein each of Sp and Z on the first ligand and on the second ligand can be the same or different.

In certain embodiments, the precursors comprise one or more metal-containing precursors and one or more chalcogen-containing precursors or pnictogen-containing precursors.

In accordance with another aspect of the present invention, there is provided a method for overcoating at least a portion of a surface of a nanoparticle according to claim 3 with a coating material having a predetermined composition, the method comprising reacting precursors for the predetermined composition in the presence of one or more chemically distinct ligands, at least of the ligands being represented by the formula:

X-Sp-Z

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle and/or (ii) a group that is cyclic, halogenated, and/or polar a-protic, wherein Z in all cases is not reactive upon exposure to light.

In certain embodiments, Z does not render the nanoparticle dispersible in a liquid medium that includes water.

In certain embodiments, a reactive group can comprise a functional, bifunctional, or polyfunctional reagent, and/or a reactive chemical group.

In certain embodiments, a cyclic group can comprise a saturated or unsaturated cyclic (including a single ring, a bicyclic structure, a multi-cyclic structure) compound or aromatic compound. In certain embodiments, the cyclic group can include at least one hetero-atom. In certain embodiments, the cyclic group can include at least one substituent group (including, for example a reactive chemical group, an organic group (alky, aryl). Other examples of cyclic groups are provided herein.

In certain embodiments, a halogenated group can comprise a fluorinated group, perfluorinated group, a chlorinated group, a perchlorinated group, a brominated group, a perbrominated group, an iodinated group, a periodinated group, etc. Other examples of halogenated groups are provided herein.

In certain embodiments, a polar a-protic group can comprise a ketone, aldehyde, amide, urea, urethane, or an imine. Other examples of polar a-protic groups are provided herein.

In certain embodiments, the nanoparticle comprises a semiconductor material.

In certain embodiments, the nanoparticle can comprise a core comprising a first material and a shell (or coating material) disposed over at least a portion of a surface of the core, the shell comprising a second material. In certain embodiments, the first material comprises a semiconductor material. In certain embodiments, the second material comprises a semiconductor material. In certain embodiments, one or more additional shells are disposed over at least a portion of a surface of the shell.

In certain preferred embodiments, a nanoparticle comprises a semiconductor nanocrystal. In certain embodiments, the semiconductor nanocrystal can comprise a core comprising a first material and a shell (or coating material) disposed over at least a portion of a surface of the core, the shell comprising a second material. Preferably, the second material comprises a nanocrystalline semiconductor material. In certain embodiments, one or more additional shells are disposed over at least a portion of a surface of the shell.

In certain embodiments, the predetermined composition of the coating material comprises a semiconductor material, preferably a nanocrystalline semiconductor material.

In certain preferred embodiments, the predetermined composition comprises one or more metals and one or more chalcogens or pnictogens

In certain embodiments, precursors include one or more metal-containing precursors and one or more chalcogen-containing or pnictogen-containing precursors.

Preferred ligands include benzylphosphonic acid, benzylphosphonic acid including at least one substituent group on the ring of the benzyl group, a conjugate base of such acids, and mixtures including one or more of the foregoing. In certain embodiments, a ligand comprises 4-hydroxybenzylphosphonic acid, a conjugate base of the acid, and a mixture of the foregoing. In certain embodiments, a ligand comprises 3, 5-di-*tert*-butyl-4-hydroxybenzylphosphonic acid, a conjugate base of the acid, or a mixture of the foregoing.

Other preferred ligands include ligands being represented by the formula X-Sp-Z comprising an organic amine including a terminal hydroxyl group or a fluorinated organic amine.

In certain embodiments, the precursors are reacted in the presence of two or more chemically distinct ligands, at least one of said ligands being represented by the formula:

X-Sp-Z,

wherein X, Sp, and Z are as described herein.

In certain embodiments, the precursors are reacted in the present of two or more chemically distinct ligands, wherein a first ligand is represented by the formula:

N-Sp-Z

wherein N represents a primary amine group, a secondary amine group, an amide group; and
a second ligand is represented by the formula:

Y-Sp-Z

wherein Y represents a phosphonic or arsonic acid group, a phosphinic or arsinic acid group, an arsenate group, a phosphine or arsine oxide group; and
wherein Sp, and Z are as described herein, and wherein each of Sp and Z on the first ligand and on the second ligand can be the same or different.

In another aspect of the present disclosure, there is provided a method for preparing nanocrystals comprising a semiconductor material, the method comprising heating a mixture comprising a liquid medium and semiconductor material precursors in the presence of a first ligand compound including an amine group (e.g., N-Sp-Z, wherein N represents a primary amine group, a secondary amine group, an imidizole group, an amide group) and a second ligand compound including an acid group (e.g., Y-Sp-Z, wherein Y represents a phosphonic or arsonic acid group, a phosphinic or arsinic acid group, a phosphate or arsenate group, a phosphine or arsine oxide group). Sp and Z are as described herein.

In certain preferred embodiments the first ligand compound including an amine group and second ligand compound including an acid group are initially present in an equimolar amount, the equimolar amount being determined based on the amine group content of the first ligand compound and the acid group content of the second ligand compound.

In certain embodiments, the first ligand compound is represented by the formula A-L, wherein A is the acid group and L comprises an aryl group, a heteroaryl group, or a straight or branched $C_{1-18}$ hydrocarbon chain. In certain embodiments, the second ligand compound is represented by the formula N-L, wherein N is the amine group and L comprises an aryl group, a heteroaryl group, or a straight or branched $C_{1-18}$ hydrocarbon chain. In certain embodiments, the hydrocarbon chain includes at least one double bond, at least one triple bond, or at least one double bond and one triple bond. In certain embodiments, the hydrocarbon chain is interrupted by -O-, - S-, $-N(R_a)-$, $-N(R_a)-C(O)-O-$, $-O-C(O)-N(R_a)-$, $-N(R_a)-C(O)-N(R_b)-$, -O-C(O)-O-, $-P(R_a)-$, or - $P(O)(R_a)-$, wherein each of $R_a$ and $R_b$ independently, is hydrogen, alkyl, alkenyl, alkynyl, alkoxy, hydroxylalkyl, hydroxyl, or haloalkyl. In certain embodiments, the aryl group is a substituted or unsubstituted cyclic aromatic group. In certain embodiments, the aryl group includes phenyl, benzyl, naphthyl, tolyl, anthracyl, nitrophenyl, or halophenyl. In certain embodiments, the heteroaryl group comprises an aryl group with one or more heteroatoms in the ring, for instance furyl, pyridyl, pyrrolyl, phenanthryl. In certain embodiments, A comprises a phosphinic acid group or a carboxylic acid group. In certain embodiments, A comprises an oleic acid group or a myristic acid group. In certain preferred embodiments, A comprises a phosphonic acid group.

In certain embodiments of the methods described herein, the method is carried out in a liquid medium. Preferably, the liquid medium comprises a coordinating solvent or mixture of coordinating solvents. Examples of coordinating solvents including those provided herein. Other coordinating solvents can also be used. In certain embodiments, the method can be carried out in a liquid medium comprising a non-coordinating solvent or mixture of non-coordinating solvents. Examples of non-coordinating solvents include, but are not limited to, squalane, octadecane, or any other saturated hydrocarbon

molecule. Mixtures of two or more solvents can also be used. Other suitable non-coordinating solvents can be readily ascertained by one of ordinary skill in the art.

In certain aspects and embodiments of the disclosure described or contemplated by this general description, the following detailed description, and claims, a nanoparticle can include a core and an overcoating (also referred to herein as a shell). A nanoparticle including a core and shell is also referred to as having a core/shell structure. The shell is disposed over at least a portion of the core. In certain embodiments, the shell is disposed over all or substantially all of the outer surface of the core. In certain embodiments of a nanoparticle including a core/shell structure, the core can comprise a first semiconductor material and the shell can comprise a second semiconductor material. In certain embodiments, the core comprises a semiconductor nanocrystal. In certain embodiments, a nanocrystal can have a diameter of less than about 10 nanometers. In embodiments including a plurality of nanoparticles, the distribution of nanoparticles sizes is preferably monodisperse.

In certain aspects and embodiments of the disclosure described or contemplated by this general description, the following detailed description, and claims, a nanoparticle is water insoluble or not dispersible in a liquid medium comprising water.

In certain aspects and embodiments of the disclosure described or contemplated by this general description, the following detailed descripion, and claims, a nanoparticle comprising a semiconductor material (preferably, a semiconductor nanocrystal) is at least partially overcoated with a coating in the presence of one or more of the ligands taught herein. In certain embodiments, the coating comprises more than one material. In certain embodiments including a coating comprising more than one material, the materials are applied sequentially. In certain embodiments, a core can include multiple overcoats or shells disposed on a surface thereof. Each of the multiple overcoats or shells can comprise the same or different composition. In certain aspects and embodiments of the disclosure described or contemplated by this general description, the following detailed description, and claims, a method is carried out in a non-aqueous medium. In certain preferred embodiments, the method is a colloidal synthesis method.

The foregoing, and other aspects and embodiments described herein all constitute embodiments of the present disclosure disclosure.

## BRIEF DESCRIPTION OF THE DRAWINGS

In the drawings:

FIGURE 1 represents the chemical structures of examples of certain compositions useful in carrying out the present invention.
FIGURE 2 depicts Atomic Force Microscope images showing 5 nm CBP thermally evaporated on an aliphatic ligand quantum dot monolayer.
FIGURE 3 depicts Atomic Force Microscope images showing 15 nm CBP thermally evaporated on an aliphatic ligand quantum dot monolayer.
FIGURE 4 depicts Atomic Force Microscope images showing 5 nm CBP thermally evaporated on an aromatic ligand quantum dot monolayer.
FIGURE 5 depicts Atomic Force Microscope images showing the effect of ligand composition on semiconductor nanocrystal layer interface morphology.
FIGURE 6 represents the chemical structures of examples of certain compositions useful in carrying out the present invention.
FIGURE 7 represents the chemical structures of examples of certain compositions useful in carrying out the present invention.
FIGURE 8 depicts spectra to illustrate a method for measuring quantum efficiency.

The attached figures are simplified representations presented for purposes of illustration only; the actual structures may differ in numerous respects, including, e.g., relative scale.

For a better understanding to the present invention, together with other advantages and capabilities thereof, reference is made to the following disclosure and appended claims in connection with the above-described drawings.

## DETAILED DESCRIPTION OF THE INVENTION

In accordance with certain embodiments of the present disclosure there is provided a nanoparticle including a ligand attached to a surface thereof, the ligand being represented by the formula:

X-Sp-Z

wherein:

X represents a primary amine group, a secondary amine group, a urea, a thiourea, an amide group, a phosphonic or arsonic acid group, a phosphinic or arsinic acid group, an arsenate group, a phosphine oxide or arsine oxide group;
Sp represents a spacer group, such as a group capable of allowing a transfer of charge or an insulating group; and
Z represents: (i) a reactive group capable of communicating specific chemical properties to the nanocrystal as well as provide specific chemical reactivity to the surface of the nanocrystal, and/or (ii) a group that is cyclic, halogenated, or polar a-protic. The ligand attaches or coordinates to a surface of the nanoparticle during formation or overcoating thereof.

Examples of Sp include straight or branched $C_1$-$C_{18}$ hydrocarbon chains. In certain embodiments, the hydrocarbon chain includes at least one double bond, at least one triple bond, or at least one double bond and one triple bond. In certain embodiments, the hydrocarbon chain is interrupted by -O-, -S-, -N($R_a$)-, -N($R_a$)-C(O)-O-, -O-C(O)-N($R_a$)-, -N($R_a$)-C(O)-N($R_b$)-, -O-C(O)-O-, -P(Ra)-, or -P(O)($R_a$)-, wherein each of $R_a$ and $R_b$, independently, is hydrogen, alkyl, alkenyl, alkynyl, alkoxy, hydroxylalkyl, hydroxyl, or haloalkyl.
Examples of reactive groups include functional, bifunctional, and polyfunctional reagents (e.g., homobifunctional or heterobifunctional), and reactive chemical groups (e.g., thiol, or carboxyl, hydroxyl, amino, amine, sulfo). Examples of additional reactive groups include carbodithioate, carbodithioic acid, thiourea, amide, phosphine oxide, phosphonic or phosphinic acid, thiophosphonic or thiophosphinic acid, which can be substituted with alkyl and/or aryl units that are perhalogenated or partially halogenated. Examples of cyclic groups include saturated or unsaturated cyclic or bicyclic compounds (e.g. cyclohexyl, isobornyl), or aromatic compounds (e.g. phenyl, benzyl, naphthyl, biphenyl, fluorenyl, triarylamine). In certain embodiments, a cyclic group can include one or more substituent groups (including, for example a reactive chemical group, an organic group (alky, aryl). Halogenated groups include fluorinated groups, perfluorinated groups, (e.g. perfluoroalkyl, perfluorophenyl, perfluoroamines), chlorinated groups, perchlorinated groups. Examples of polar a-protic groups include ketones, aldehydes, amides, ureas, urethanes, imine,
**[0078]** In certain embodiments, the group comprising Z imparts predetermined chemical miscibility properties to the semiconductor nanocrystal to which it is attached.
In certain embodiments, Z does not include a non-functionalized straight or branched $C_1$-$C_{18}$ hydrocarbon chain.
In certain embodiments, the nanoparticle comprises a semiconductor nanoparticle. In certain preferred embodiments, the nanoparticle comprises a semiconductor nanocrystal.
In accordance with certain embodiments, there is provided a nanoparticle including two or more chemically distinct ligands attached to a surface thereof, at least one of said ligands being represented by the formula:

X-Sp-Z

wherein:

X represents a primary amine group, a secondary amine group, a urea, a thiourea, an amide group, phosphonic or arsonic acid group, a phosphinic or arsinic acid group, an arsenate group, a phosphine oxide or arsine oxide group;
Sp represents a spacer group, such as a group capable of allowing a transfer of charge or an insulating group; and
Z represents: (i) a reactive group capable of communicating specific chemical properties to the nanocrystal as well as provide specific chemical reactivity to the surface of the nanocrystal, and/or (ii) a group that is cyclic, halogenated, or polar a-protic.

Examples of Sp and Z include those described herein.
In certain embodiments, Z does not include a non-functionalized straight or branched $C_1$-$C_{18}$ hydrocarbon chain.
In certain embodiments, the nanoparticle comprises a semiconductor nanoparticle. In certain preferred embodiments, the nanoparticle comprises a semiconductor nanocrystal.
In accordance with certain embodiments there is provided a nanoparticle including two or more chemically distinct ligands attached to a surface thereof, wherein a first ligand is represented by the formula:

N-Sp-Z

wherein:

N represents a primary amine group, a secondary amine group, a urea, a thiourea, an amide group, or other nitrogen containing functional group;
Sp represents a spacer group, such as a group capable of allowing a transfer of charge or an insulating group; and

Z represents a reactive group capable of communicating specific chemical properties to the nanocrystal and/or provide specific chemical reactivity to the surface of the nanocrystal; and

a second ligand is represented by the formula:

Y-Sp-Z

wherein:

Y represents a phosphonic or arsonic acid group, a phosphinic or arsinic acid group, an arsenate group, a phosphine or arsine oxide group, or other phosphorous-containing or arsenic-containing functional group;
Sp represents a spacer group, such as a group capable of allowing a transfer of charge or an insulating group; and
Z represents a reactive group capable of communicating specific chemical properties to the nanocrystal and/or provide specific chemical reactivity to the surface of the nanocrystal.

Examples of Sp and Z for inclusion in N-Sp-Z and P-Sp-Z include those described herein. Sp and Z can be independently selected. Sp and Z included in each of the two ligands can be the same or different.
In certain embodiments, Z does not include a non-functionalized straight or branched $C_1$-$C_{18}$ hydrocarbon chain.
In certain embodiments, the nanoparticle comprises a semiconductor nanoparticle. In certain preferred embodiments, the nanoparticle comprises a semiconductor nanocrystal.
In accordance with another embodiment of the disclosure there is provided a method for functionalizing a nanoparticle. The method comprise reacting precursors for forming a nanoparticle having a predetermined composition in the presence of the herein described ligand X-Sp-Z, wherein X, Sp, and Z are as described herein.
In certain embodiments, the nanoparticle comprises a semiconductor nanoparticle. In certain preferred embodiments, the nanoparticle comprises a semiconductor nanocrystal. In certain embodiments, the predetermined precursors include a metal-containing precursor and a chalcogen-containing or pnictogen-containing precursor. The precursors are preferably included in the reaction mixture in amounts based on the predetermined composition.
In certain embodiments, the method is carried out in a liquid medium. Preferably, the liquid medium comprises a coordinating solvent or mixture of coordinating solvents. Examples of coordinating solvents including those provided herein. Other coordinating solvents can also be used. In certain embodiments, the method can be carried out in a liquid medium comprising a non-coordinating solvent or mixture of non-coordinating solvents. Examples of non-coordinating solvents include, but are not limited to, squalane, octadecane, or any other saturated hydrocarbon molecule. Mixtures of two or more solvents can also be used. Other suitable non-coordinating solvents can be readily ascertained by one of ordinary skill in the art.
In certain embodiments, the mole ratio of total metal included in the one or more metal-containing precursors to total moles of ligand represented by the formula X-Sp-Z is in the range from about 1: 0.1 to about 1:100. In certain embodiments, the mole ratio of total moles of metal included in the one or more metal-containing precursors to total moles of ligand represented by the formula X-Sp-Z is in the range from about 1: 1 to about 1:50. In certain embodiments, the mole ratio of total moles of metal included in the one or more metal-containing precursors to total moles of ligand represented by the formula X-Sp-Z is in the range from about 1:1 to about 1:30. In certain embodiments in which the method is carried out in a liquid medium, the mole ratio of total moles of the liquid medium to total moles of ligand represented by the formula X-Sp-Z is in the range from about 500:1 to about 2:1. In certain embodiments, the mole ratio of total moles of the liquid medium to total moles of ligand represented by the formula X-Sp-Z is in the range from about 100:1 to about 5:1. In certain embodiments, the mole ratio of total moles of the liquid medium to total moles of ligand represented by the formula X-Sp-Z is in the range from about 50:1 to about 5:1.
In accordance with certain embodiments, there is provided a method for functionalizing a nanoparticle. The method comprises reacting precursors for forming a nanoparticle having a predetermined composition in the presence of two or more chemically distinct ligands, at least one of said ligands being represented by the formula:

X-Sp-Z

wherein:

X represents a primary amine group, a secondary amine group, a urea, a thiourea, an amide group, phosphonic or arsonic acid group, a phosphinic or arsinic acid group, an arsenate group, a phosphine oxide or arsine oxide group;
Sp represents a spacer group, such as a group capable of allowing a transfer of charge or an insulating group; and
Z represents: (i) a reactive group capable of communicating specific chemical properties to the nanocrystal as well as provide specific chemical reactivity to the surface of the nanocrystal, and/or (ii) a group that is cyclic, halogenated,

or polar a-protic.

In certain embodiments, Z does not include a non-functionalized straight or branched $C_1$-$C_{18}$ hydrocarbon chain. Examples of Sp and Z include those described herein.
In certain embodiments, the nanoparticle comprises a semiconductor nanoparticle. In certain preferred embodiments, the nanoparticle comprises a semiconductor nanocrystal. In certain embodiments, the predetermined precursors include a metal-containing precursor and a chalcogen-containing or pnictogen-containing precursor. The precursors are preferably included in the reaction mixture in amounts based on the predetermined composition.
In certain embodiments there is provided a method for functionalizing a nanoparticle. The method comprises reacting precursors for forming a nanoparticle having a predetermined composition in the presence of two or more chemically distinct ligands, wherein a first ligand is represented by the formula:

N-Sp-Z

wherein:

N represents a primary amine group, a secondary amine group, a urea, a thiourea, an amide group;
Sp represents a spacer group, such as a group capable of allowing a transfer of charge or an insulating group; and
Z represents a reactive group capable of communicating specific chemical properties to the nanocrystal as well as provide specific chemical reactivity to the surface of the nanocrystal; and

a second ligand is represented by the formula:

Y-Sp-Z

wherein:

Y represents phosphonic or arsonic acid group, a phosphinic or arsonic acid group an arsenate group, a phosphine or arsine oxide group;
Sp represents a spacer group, such as a group capable of allowing a transfer of charge or an insulating group; and
Z represents a reactive group capable of communicating specific chemical properties to the nanocrystal as well as provide specific chemical reactivity to the surface of the nanocrystal.

Examples of Sp and Z include those described herein. Sp and Z can be independently selected. Sp and Z included in each of the two ligands can be the same or different.
In certain embodiments, the nanoparticle comprises a semiconductor nanoparticle. In certain preferred embodiments, the nanoparticle comprises a semiconductor nanocrystal. In certain embodiments, the predetermined precursors include a metal-containing precursor and a chalcogen-containing or pnictogen-containing precursor. The precursors are preferably included in the reaction mixture in amounts based on the predetermined composition.In accordance another embodiment of the invention, there is provided a method for overcoating at least a portion of a surface of a nanoparticle with a coating material having a predetermined composition, the method comprising reacting precursors for the predetermined coating material in the presence of a ligand represented by the formula X-Sp-Z and the nanoparticle to be coated, wherein X, Sp, and Z are as described herein. In certain embodiments, the nanoparticle comprises a semiconductor nanoparticle. In certain preferred embodiments, the nanoparticle comprises a semiconductor nanocrystal. In certain embodiments, the coating composition comprises a semiconductor material. In certain embodiments, the precursors include a metal-containing precursor and a chalcogen-containing or pnictogen-containing precursor. The precursors are preferably included in the reaction mixture in amounts based on the predetermined composition.
In certain embodiments, the method is carried out in a liquid medium. Preferably, the liquid medium comprises a coordinating solvent or mixture of coordinating solvents. Examples of coordinating solvents including those provided herein. Other coordinating solvents can also be used. In certain embodiments, the method can be carried out in a liquid medium comprising a non-coordinating solvent or mixture of non-coordinating solvents. Examples of non-coordinating solvents include squalane, octadecane, or any other saturated hydrocarbon molecule. Mixtures of two or more solvents can also be used. Other suitable non-coordinating solvents can be readily ascertained by one of ordinary skill in the art.
In certain embodiments, the mole ratio of total metal included in the nanoparticles being overcoated to total moles of ligand represented by the formula X-Sp-Z is in the range from about 1: 0.1 to about 1:100. In certain embodiments, the mole ratio of total moles of metal included in the nanoparticles being overcoated to total moles of ligand represented by the formula X-Sp-Z is in the range from about 1: 1 to about 1:50. In certain embodiments, the mole ratio of total moles of metal included in the nanoparticles being overcoated to total moles of ligand represented by the formula X-Sp-Z is in

the range from about 1:1 to about 1:30.

In certain embodiments of the method being carried out in a liquid medium, the mole ratio of total moles of the liquid medium to total moles of ligand represented by the formula X-Sp-Z is in the range from about 500:1 to about 2:1. In certain embodiments, the mole ratio of total moles of the liquid medium to total moles of ligand represented by the formula X-Sp-Z is in the range from about 100:1 to about 5:1. In certain embodiments, the mole ratio of total moles of the liquid medium to total moles of ligand represented by the formula X-Sp-Z is in the range from about 50:1 to about 5:1.In accordance with further embodiments of the disclosure there is provided a method for overcoating at least a portion of a surface of a nanoparticle with a coating material having a predetermined composition, the method comprising reacting precursors for the predetermined coating material in the presence of two or more chemically distinct ligands and the nanoparticle to be coated, at least one of said ligands being represented by the formula:

X-Sp-Z

and the nanoparticle to be coated, wherein:

X represents a primary amine group, a secondary amine group, a urea, a thiourea, an amide group, phosphonic or arsonic acid group, a phosphinic or arsinic acid group, an arsenate group, a phosphine oxide or arsine oxide group;
Sp represents a spacer group, such as a group capable of allowing a transfer of charge or an insulating group; and
Z represents: (i) a reactive group capable of communicating specific chemical properties to the nanocrystal as well as provide specific chemical reactivity to the surface of the nanocrystal, and/or (ii) a group that is cyclic, halogenated, or polar a-protic.

In certain embodiments, Z does not include a non-functionalized straight or branched $C_1$-$C_{18}$ hydrocarbon chain.

Examples of Sp and Z include those described herein.

In certain embodiments, the nanoparticle comprises a semiconductor nanoparticle. In certain preferred embodiments, the nanoparticle comprises a semiconductor nanocrystal. In certain embodiments, the coating composition comprises a semiconductor material. In certain embodiments, the precursors include a metal-containing precursor and a chalcogen-containing or pnictogen-containing precursor. The precursors are preferably included in the reaction mixture in amounts based on the predetermined composition.

In certain embodiments, there is provided a method for overcoating at least a portion of a surface of a nanoparticle with a coating material having a predetermined composition, the method comprising reacting precursors for the predetermined coating material in the presence of two or more chemically distinct ligands and the nanoparticle to be coated, wherein a first ligand is represented by the formula:

N-Sp-Z

wherein:

N represents a primary amine group, a secondary amine group, a urea, a thiourea, an amide group;
Sp represents a spacer group, such as a group capable of allowing a transfer of charge or an insulating group; and
Z represents a reactive group capable of communicating specific chemical properties to the nanocrystal as well as provide specific chemical reactivity to the surface of the nanocrystal; and

a second ligand is represented by the formula:

Y-Sp-Z

wherein Y represents a phosphonic or arsonic acid group, a phosphinic or arsinic acid group, an arsenate group, a phosphine or arsine oxide group;
Sp represents a spacer group, such as a group capable of allowing a transfer of charge or an insulating group; and
Z represents a reactive group capable of communicating specific chemical properties to the nanocrystal as well as provide specific chemical reactivity to the surface of the nanocrystal.

Examples of Sp and Z include those described herein. Sp and Z can be independently selected. Sp and Z including in the two ligands can be the same or different.

In certain embodiments, the nanoparticle comprises a semiconductor nanoparticle. In certain preferred embodiments, the nanoparticle comprises a semiconductor nanocrystal.

In certain embodiments, the coating composition comprises a semiconductor material. In certain embodiments,

the precursors include a metal-containing precursor and a chalcogen-containing or pnictogen-containing precursor. The precursors are preferably included in the reaction mixture in amounts based on the predetermined composition.

In carrying out the methods described herein, the precursors are selected and reacted in amounts and under reaction conditions, and for a period of time, to produce a nanoparticle having the predetermined composition. Such variables can be routinely determined by a person of ordinary skill in the relevant art. In certain embodiments, the reaction is carried out in a controlled atmosphere (substantially free of water moisture and air). In certain preferred embodiments, the reaction is carried out in a water-free inert atmosphere.

In certain embodiments of the present disclosure, a nanoparticle (e.g., a semiconductor nanocrystal) is formed, or overcoated in order to generate a shell on at least a portion of an outer surface of a nanoparticle, in the presence of at least one molecule having the following formula:

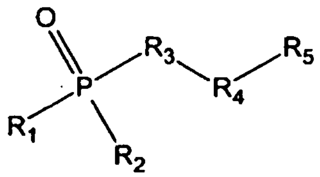 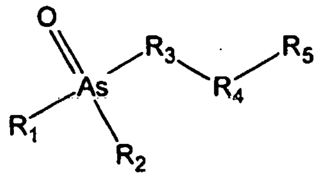 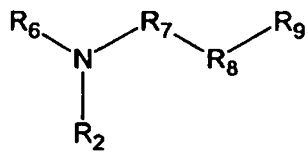

Wherein $R_1$ represents a hydroxyl group; $R_2$ represents a hydroxyl, hydrogen, an alkyl or alkylene group, an aryl or arylene group, $-OR_{11}$, $-NHR_{11}$, $-NR_{11}R_{11}$, $-SR_{11}$, wherein $R_{11}$ represents hydrogen, an alkyl group, or an aryl group; $R_3$ and $R_4$, which can be the same or different, represent a bond, an alkyl or alkylene group, an aryl or arylene group, a fluorocarbon group, -O- -NH- -S- $-OR_{12}-$ $-NR_{12}-$

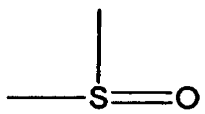 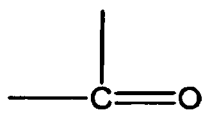

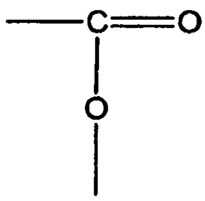 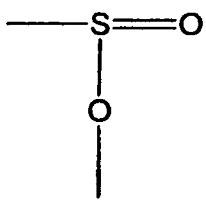 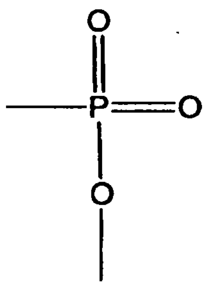 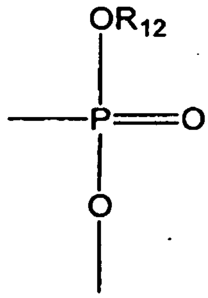

wherein $R_{12}$ is an alkyl or alkylene group or an aryl or arylene group; $R_5$ represents hydrogen, an alkyl group including one or more functional groups, an alkylene group, an aryl or arylene group, $-OR_{13}$, $-NHR_{13}$, $-NR_{13}R_{13}$, $-SR_{13}$, wherein $R_{13}$ represents hydrogen, an alkyl group, or an aryl group; $R_6$ represents hydrogen; $R_7$ represents hydrogen, an alkyl or alkylene group, an aryl or arylene group, $-OR_{14}$, $-NHR_{14}$, $-NR_{14}R_{14}$, $-SR_{14}$, wherein $R_{14}$ represents hydrogen, an alkyl group, or an aryl group; $R_8$ and $R_9$, which can be the same or different, represent a bond, an alkylene group, an aryl or arylene group, a fluorocarbon group,- O- -NH- -S- $-OR_{15}-$ $-NR_{15}-$

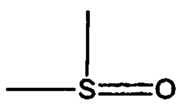 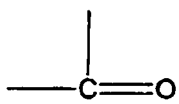

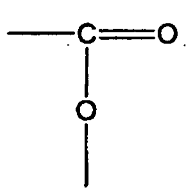

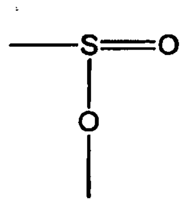

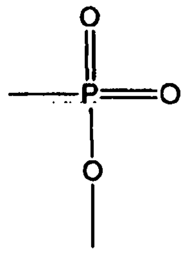

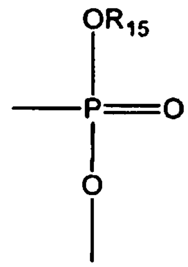

wherein $R_{15}$ is an alkyl or alkylene group or an aryl or arylene group; $R_8$ can also represent an alkyl group; ; $R_9$ can represent an alkyl group including one or more functional groups; $R_{10}$ represents hydrogen, an alkyl or alkylene group, an aryl or arylene group, $-OR_{16}$, $-NHR_{16}$,$-NR_{16}R_{16}$, $-SR_{16}$, wherein $R_{16}$ represents hydrogen, an alkyl group, or an aryl group.

Furthermore, the architecture described herein opens up the possibility for a modular synthetic scheme for tailoring a quantum dot surface with any desired characteristic. For example, a terminal hydroxyl group provides a site for additional chemical reactivity. The nucleophilic nature of the -OH group can do various addition and substitution reactions with the appropriate electrophile. For example, quantum dots (e.g., semiconductor nanocrystals) with this nucleophilic surface group can be reacted with an electrophile, such as an acid chloride, isocyanate, or carboxylic acid group resulting in the following ester and urethane linkages:

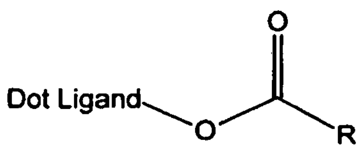

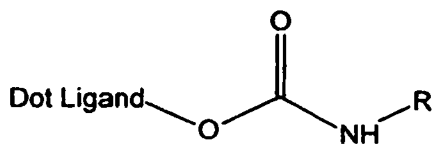

Where R can be any substituent. The -OH group can also be substituted for a primary amine resulting in the following amide and urea linkages with the electrophiles mentioned herein:

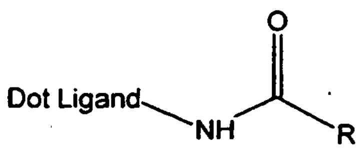

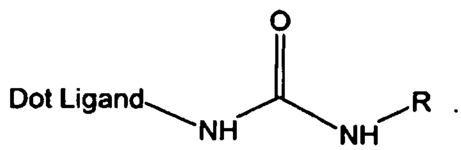

Where, again, R can be any substituent.

***Functionalizing surface of the semiconductor nanocrystal with terminal hydroxyl groups.***

**Chemical Structures used:**

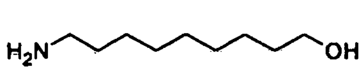

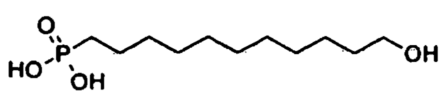

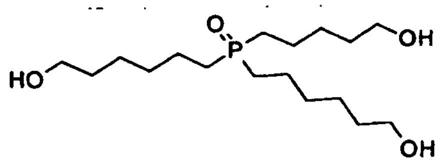

This approach may be achieved in the presence of phosphine oxide (TOPO) with the addition of high boiling polar a-protic solvents (e.g. 1,3-Dimethyl-2-imidazolidone (DMI), Carbitol Acetate, N,N-Dimethylacrylamide (DMAc), 1-Methyl-2-pyrrolidnone (NMP), etc.).

***Examples of coupling species to hydroxy-terminated semiconductor nanocrystals.***

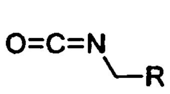 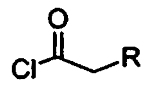 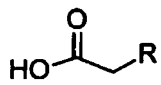

The functional groups are isocyanates, acid chlorides, and carboxylic acids from left to right. Where R can be, and is most certainly not limited to, any of the following chemical species (with any length aliphatic chain linker connecting the molecule to the functional group shown herein).

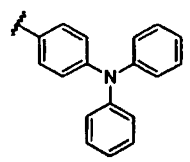 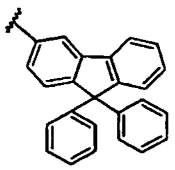 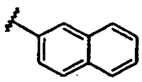 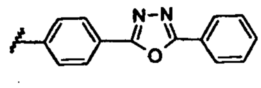 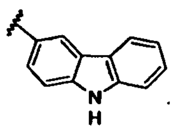

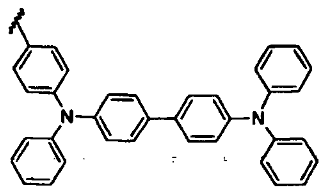 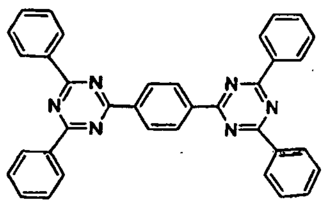 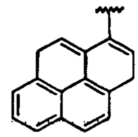 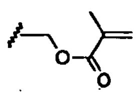 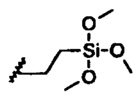

The system described herein can be further augmented by building similar variability into the phosphine oxide derivative used as the solvent in the overcoating procedure. This species has the formula:

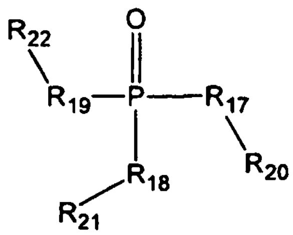 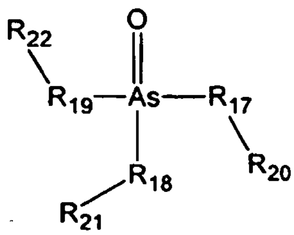

Wherein $R_{17}$, $R_{18}$, and $R_{19}$, which can be the same or different, represent a bond, an alkyl or alkylene group, an aryl or arylene group, a fluorocarbon group,

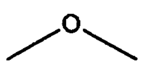 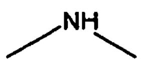 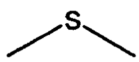 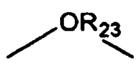

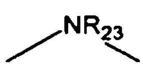 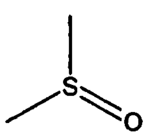 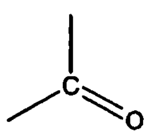

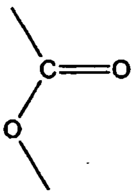 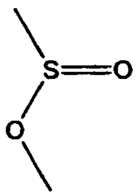 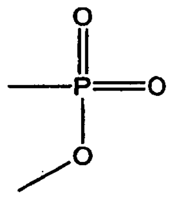 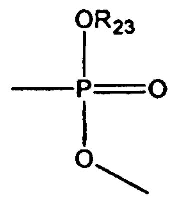

wherein $R_{23}$ is an alkyl or alkylene group or an aryl or arylene group; $R_{20}$, $R_{21}$, and $R_{22}$, which can be the same or different, represent hydrogen, an alkyl or alkylene group, an aryl or arylene group, $-OR_{24}$, $NHR_{24}$, $-NR_{24}R_{24}$, $-SR_{24}$, wherein $R_{24}$ represents hydrogen, an alkyl group, or an aryl group.

To avoid the introduction of impurities which may have an unpredictable effect on the reaction, the ligands should preferably have a purity of at least 99 wt. %, and preferably greater than 99.5%.

Phosphinic or arsinic acid groups useful in the practice of the invention may include mono- and di-phosphinic/arsinic acid groups.

In accordance with certain embodiments of the present disclosure, a nanoparticle (e.g., a semiconductor nanocrystal) is formed, or overcoated in order to generate a shell on at least a portion of an outer surface of a nanoparticle, in the presence of molecules represented by one of the following formula or in the presence of molecules of both of the following formula:

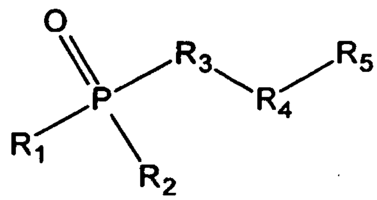 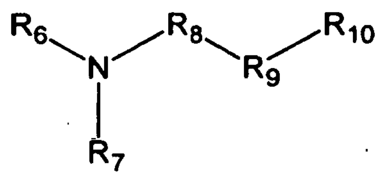

(in the above formula on the left, P can alternatively be As)

wherein $R_1$ represents a hydroxyl group; $R_2$ represents hydrogen, an alkyl or alkylene group, an aryl or arylene group, $-OR_{11}$, $NHR_{11}$, $-NR_{11}R_{11}$, $-SR_{11}$, wherein $R_{11}$ represents hydrogen, an alkyl group, or an aryl group; $R_3$ and $R_4$, which can be the same or different, represent a bond, an alkyl or alkylene group, an aryl or arylene group, a fluorocarbon group, -O- -NH- -S- $-OR_{12}-$ $-NR_{12}-$

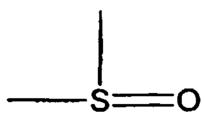 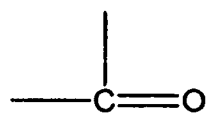

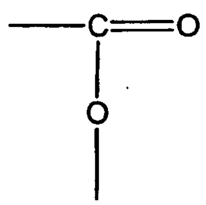 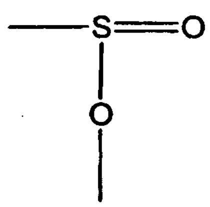 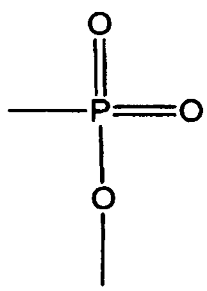 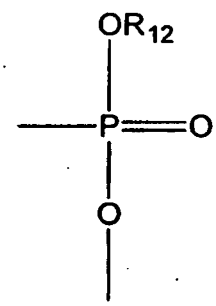

wherein $R_{12}$ is an alkyl or alkylene group or an aryl or arylene group; $R_5$ represents hydrogen, an alkyl group including one or more functional groups , an alkylene group, an aryl or arylene group, $-OR_{13}$, $-NHR_{13}$, $-NR_{13}R_{13}$, $-SR_{13}$, wherein $R_{13}$ represents hydrogen, an alkyl group, or an aryl group; $R_6$ represents hydrogen; $R_7$ represents hydrogen, an alkyl or alkylene group, an aryl or arylene group, $-OR_{14}$, $-NHR_{14}$, $-NR_{14}R_{14}$, $-SR_{14}$, wherein $R_{14}$ represents hydrogen, an alkyl group, or an aryl group; $R_8$ and $R_9$, which can be the same or different, represent a bond, an alkyl or alkylene group, an aryl or arylene group, a fluorocarbon group, -O- -NH- -S- $-OR_{15}-$ $-NR_{15}-$

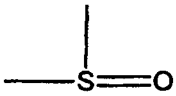 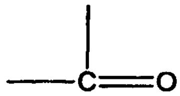

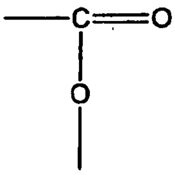 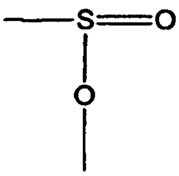 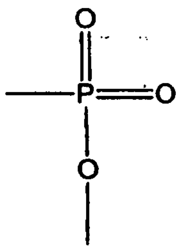 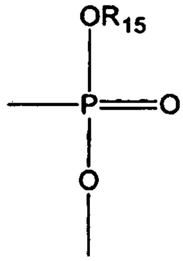

wherein $R_{15}$ is an alkyl or alkylene group or an aryl or arylene group; $R_{10}$ represents hydrogen, an alkyl group including one or more functional groups, an alkylene group, an aryl or arylene group,- $OR_{16}$, $-NHR_{16}$, $-NR_{16}R_{16}$, $-SR_{16}$, wherein $R_{16}$ represents hydrogen, an alkyl group, or an aryl group.

As mentioned herein, the system can be further augmented by building similar variability into the phosphine oxide derivative used as the solvent in the overcoating procedure. This species has the formula:

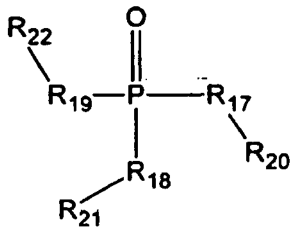

Wherein $R_{17}$, $R_{18}$, and $R_{19}$, which can be the same or different, represent a bond, an alkyl or alkylene group, an aryl or arylene group, a fluorocarbon group,

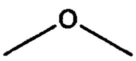 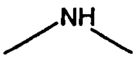 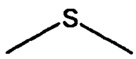 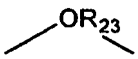

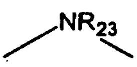 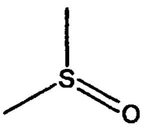 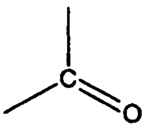

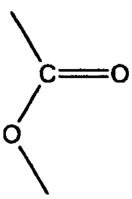 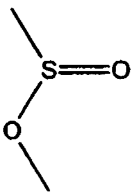 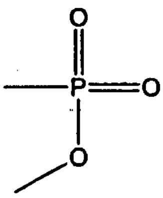 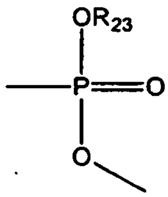

wherein $R_{23}$ is an alkyl or alkylene group or an aryl or arylene group; $R_{20}$, $R_{21}$, and $R_{22}$, which can be the same or different, represent hydrogen, an alkyl or alkylene group, an aryl or arylene group, $-OR_{24}$, $-NHR_{24}$, $-NR_{24}R_{24}$, $-SR_{24}$, wherein $R_{24}$ represents hydrogen, an alkyl group, or an aryl group.

To avoid the introduction of impurities which may have an unpredictable effect on the reaction, the ligands should preferably have a purity of at least 99 wt. %,, and preferably greater than 99.5%.

Phosphinic acid groups useful in the practice of the invention may include mono and diphosphinic acid groups.

As described herein, arsenic variations of the above-described phosphorus-containing acid and oxide groups can also be used.

The present invention will be further clarified by the following examples, which are intended to be exemplary of the present invention.

## EXAMPLES

### Example 1A

Performing the overcoating procedure with TOPO as the solvent, replacing the existing aliphatic phosphonic acid and amine species with aromatic derivatives (see Figure 1) results in semiconductor nanocrystals that have new surface chemistry while maintaining their optical properties. These semiconductor nanocrystals are no longer soluble in hexane, but are readily soluble in toluene and chloroform. In addition, thin films of organic molecules can be reliably deposited onto ordered films of these synthetically modified nanocrystals without the "puddling" associated with traditional aliphatic semiconductor nanocrystal surface chemistry (see Figures 2-5). It is believed that a phosphonic acid/amine salt is the predominant species on the surface of the semiconductor nanocrystal despite the fact that TOPO is in large excess during the reaction.

***Preparation of Aromatic Semiconductor Nanocrystals Capable of Emitting Red Light***

*Synthesis of CdSe Cores:* 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

*Synthesis of CdSe/CdZnS Core-Shell Nanocrystals:* 25.86 mmol of trioctylphosphine oxide and 2.4 mmol of benzylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure and then 2.4 mmol of phenylethylamine was added to the reaction mixture. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over the course of 2 hours at 155°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in toluene. The semiconductor nanocrystals had an emission maximum of 616 nm with a FWHM of 34 nm and a solution quantum yield of 50%.

*Sample Fabrication.* Cleaned glass substrates were ashed in a plasma preen and coated with PEDOT:PSS (70nm). Substrates were taken into a nitrogen environment and baked at 120C for 20 minutes. 50nm E105 (N,N'-Bis(3-methylphenyl)-N,N'-bis-(phenyl-9,9-spiro-bifluorene, LumTec) was evaporated in a vacuum chamber below 2e-6 Torr via thermal evaporation. Application of aromatic quantum dots was accomplished via contact printing. A dispersion of semiconductor nanocrystals with an optical density (OD) of 0.3 at the 1$^{st}$ absorption feature was spin-coated at 3000rpm on a parylene coated stamp for 60 seconds, which was then stamped onto the E105 substrates depositing a mono-layer of aromatic quantum dots. Substrates were then taken back into the thermal evaporation chamber, and 5nm and 15nm, respectively, of CBP (4,4'-Bis(carbazol-9-yl)biphenyl, LumTec) were evaporated below 2e-6 Torr. Figures 2-5 depict images of the samples described in this Sample Fabrication example.

Following examples 1-B and 1-C relate to preparing semiconductor nanocrystals including benzyl phosphonic acid ligands, but without the phenylethylamine shown in Figure 1:

**Example 1-B**

*Preparation of Semiconductor Nanocrystals Capable of Emitting Green Light*

*Synthesis of ZnSe Cores:* 0.69 mmol diethyl zinc was dissolved in 5 mL of tri-n-octylphosphine and mixed with I mL of 1 M TBP-Se. 28.9 mmol of Oleylamine was loaded into a 3-neck flask, dried and degassed at 90°C for one hour. After degassing, the flask was heated to 310°C under nitrogen. Once the temperature reached 310°C, the Zn solution was injected and the reaction mixture was heated at 270°C for 15-30 minutes while aliquots of the solution were removed periodically in order to monitor the growth of the nanocrystals. Once the first absorption peak of the nanocrystals reached 350 nm, the reaction was stopped by dropping the flask temperature to 160°C and used without further purification for preparation of CdZnSe cores.

*Synthesis of CdZnSe Cores:* 1.12 mmol dimethylcadmium was dissolved in 5 mL of tri-n-octylphosphine and mixed with 1 mL of 1 M TBP-Se. In a 4-neck flask, 41.38 mmol of trioctylphosphine oxide and 4 mmol of hexylphosphonic acid were loaded, dried and degassed at 120°C for one hour. After degassing, the oxide/acid was heated to 160°C under nitrogen and 8 ml of the ZnSe core growth solution was transferred at 160°C into the flask, immediately followed by the addition of Cd/Se solution over the course of 20 minutes via syringe pump. The reaction mixture was then heated at 150°C for 16-20 hours while aliquots of the solution were removed periodically in order to monitor the growth of the nanocrystals. Once the emission peak of the nanocrystals reached 500 nm, the reaction was stopped by cooling the mixture to room temperature. The CdZnSe cores were precipitated out of the growth solution inside a nitrogen atmosphere glovebox by adding a 2:1 mixture of methanol and n-butanol. The isolated cores were then dissolved in hexane and used to make core-shell materials.

*Synthesis of CdZnSe/CdZnS Core-Shell Nanocrystals:* 25.86 mmol of trioctylphosphine oxide and 2.4 mmol of benzylphosphonic acid were loaded into a four-neck flask. The mixture was then dried and degassed in the reaction vessel by heating to 120°C for about an hour. The flask was then cooled to 75°C and the hexane solution containing isolated CdZnSe cores (0.1 mmol Cd content) was added to the reaction mixture. The hexane was removed under reduced pressure. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 150°C under nitrogen. The precursor solutions were added dropwise over the course of 1 hour at 150°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in hexane and used to make semiconductor nanocrystal composite materials.

**Example 1-C**

*Preparation of Semiconductor Nanocrystals Capable of Emitting Red Light*

*Synthesis of CdSe Cores:* 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

*Synthesis of CdSe/CdZnS Core-Shell Nanocrystals:* 25.86 mmol of trioctylphosphine oxide and 2.4 mmol of benzylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over the course of 2 hours at 155°C using a syringe pump. After the shell growth, the

nanocrystals were transferred to a nitrogen atmosphere glovebox and precipitated out of the growth solution by adding a 3:1 mixture of methanol and isopropanol. The isolated core-shell nanocrystals were then dissolved in toluene and used to make quantum dot composite materials.

**Example 2**

Performing the overcoating procedure with TOPO as the solvent, a fluorinated derivative of the amine species was used with hexylphosphonic acid, an aliphatic phosphonic acid (see Figure 6). After the reaction, these semiconductor nanocrystals were no longer soluble in conventional organic solvents, such as hexane, toluene, chloroform, methylene chloride, etc. However, the sample was soluble in fluorinated solvents such as perfluorohexane, perfluorotoluene, and Fluorinert (FC-77). The level of fluorination could be enhanced by using the fluorinated amine with a fluorinated phosphonic acid derivative and/or a fluorinated TOPO equivalent in synthesis.

Fluorinating the surface of the semiconductor nanocrystals can facilitate deposition of the material in various applications. Fluorinated semiconductor nanocrystals have been successfully spin-cast directly onto organic thin-films since the fluorinated solvent was unable to solvate the organic transport materials.

**Example 3**

Performing the overcoating procedure with TOPO as the solvent, an amine species functionalized with a terminal hydroxyl group was implemented with hexylphosphonic acid (see Figure 7). After the reaction, the sample was not soluble in hexane or toluene but was soluble in polar solvents such as methanol and isopropanol. Again, polarity of the semiconductor nanocrystal surface could be enhanced by using an amine with an alkyl or aryl phosphonic acid with a terminal hydroxyl group and/or an alkyl or aryl phosphine oxide with a terminal hydroxyl group.

**Example 4**

*Preparation of Semiconductor Nanocrystals Capable of Emitting Red Light*

*Synthesis of CdSe Cores:* 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

*Synthesis of CdSe/CdZnS Core-Shell Nanocrystals:* 25.86 mmol of trioctylphosphine oxide and 2.4 mmol of octadecylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure and then 2.4 mmol of 6-amino-1-hexanol was added to the reaction mixture. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over the course of 2 hours at 155°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in hexane.

***Preparation of Layer including Semiconductor Nanocrystals***

Films listed in Table 1 below are prepared using samples including semiconductor nanocrystals prepared substantially in accordance with one of the above-described examples dispersed in hexane. (A sample typically represents approximately 40 mg of solid dispersed in 10-15 ml hexane.) The hexane is removed from the semiconductor nanocrystals under vacuum at room temperature. Care is taken not to overdry or completely remove all solvent. 0.5ml

of RD-12, a low viscosity reactive diluent commercially available from Radcure Corp, 9 Audrey Pl, Fairfield, NJ 07004-3401, United States, is added to the semiconductor nanocrystals while stirring magnetically. After the semiconductor nanocrystals are pre-solubilized in the reactive diluent, 2ml of DR-150, UV-curable acrylic formulation commercially available Radcure, is added dropwise while stirring vigorously. Occasionally, the mixing vial is heated to lower viscosity and aid stirring. After the addition is competed, vacuum is pulled to remove entrained air. The vial is then placed in an ultrasonic bath (VWR) from 1 hour to overnight, resulting in a clear, colored solution. Care is taken to avoid temperatures over 40C while the sample is in the ultrasonic bath.

Multiple batches of the semiconductor nanocrystals of the same color in UV curable acrylic are mixed together. For the samples below, the three red batches listed in Table 1 were added together; and four green batches listed in Table I were added together.

Samples are coated by Mayer rod on precleaned glass slides and cured in a 5000-EC UV Light Curing Flood Lamp from DYMAX Corporation system with an H-bulb (225 mW/cm$^2$) for 10 seconds.

Samples including multiple layers for achieving the desired thickness are cured between layers. Samples including filters on top of (or below) the semiconductor nanocrystal/matrix layers have the filters coated by Mayer rod in a separate step. Filters are made by blending UV-curable pigment ink formulations from Coates/Sun Chemical. A filter composition is formulated by adding the weighted absorbances of the individual colors together to achieve the desired transmission characteristics.

**Table** 1

| **Film Color/Batch # (Nanocrystal Prep. Example #)** | **Solvent** | **Ligand(s)** | **Emission (nm)** | **FWHM** | **Solution QY (%)** |
|---|---|---|---|---|---|
| Red/Batch #1 (Ex. 4) | Hexane | ODPA with 6-amino-1-hexanol | 617 | 40 | 73 |
| Red/Batch # 2 (Ex. 4) | Hexane | ODPA with 6-amino-1-hexanol | 622 | 44 | 82 |
| Red/Batch #3 (Ex. 4) | Hexane | ODPA with 6-amino-1-hexanol | 624 | 44 | 73 |
| Green/Batch #1 (Ex 1A) | Hexane | Aromatic | 525 | 34 | 68 |
| Green/Batch #2 (Ex 1A) | Hexane | Aromatic | 527 | 34 | 66 |
| Green/Batch #3 (Ex 1A) | Hexane | Aromatic | 528 | 36 | 64. |
| Green/Batch #4 (Ex 1A) | Hexane | Aromatic | 530 | 33 | 60 |
| Green/Batch #5 (Ex 1A) | Hexane | Aromatic | 529 | 33 | 68 |

Examples of other variations for synthesizing semiconductor nanocrystals with aromatic surface functionality include the following. The overcoating process can be carried out in the absence of any ligand with an aliphatic group. In other words, the procedure can be performed without trioctylphosphine oxide (TOPO) or trioctylphosphine (TOP) and instead use a non-coordinating solvent (e.g., squalane). In order to maintain solubility of the semiconductor nanocrystals made in this process, multiple distinct aromatic phosphonic acid species and/or multiple distinct aromatic amine species may be included in the reaction in order to break-up crystallization or ordered packing of ligand species (both intra-semiconductor nanocrystal and inter-semiconductor nanocrystal) and allow the semiconductor nanocrystals to be dispersed in various solvent systems. Alternatively, branched phosphonic acids and/or branched amines can be used for this purpose.

**Example 5**

***Preparation of Semiconductor Nanocrystals Capable of Emitting Red Light with 3,5-di-tert-butyl-4-hydroxybenzylphosphonic acid***

***Synthesis of CdSe Cores***: 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought

back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

***Preparation of 3,5-Di-tert-butyl-4-hydroxybenzylphosphonic acid***

3,5-Di-tert-butyl-4-hydroxybenzylphosphonic acid was obtained from PCI Synthesis, 9 Opportunity Way, Newburyport, Massachusetts 01950.

The preparation of 3,5-Di-tert-butyl-4-hydroxybenzylphosphonic acid utilized the following synthetic approach:

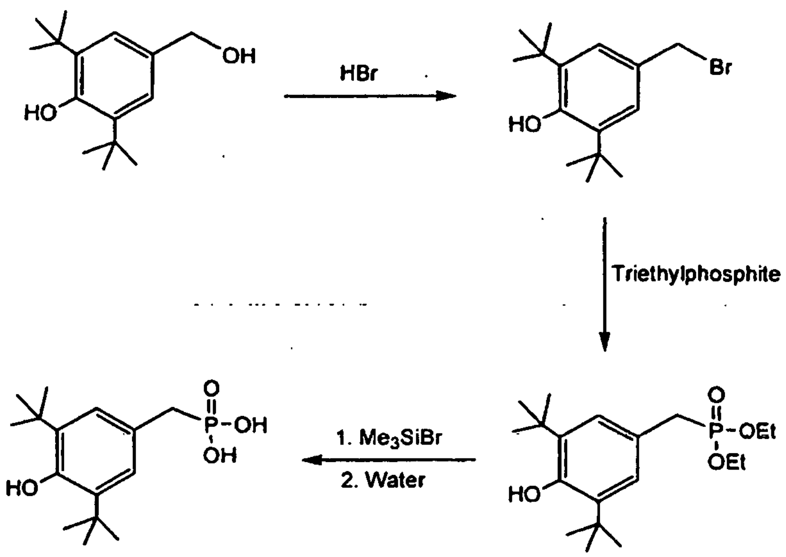


3,5-Di-tert-butyl-4-hydroxybenzylphosphonic acid can be characterized by the following::

Melting point: 199-200°C [Lit: 200°C; Literature ref: J.D. Spivack, FR1555941 (**1969**)] IR: 3614cm$^{-1}$, 3593cm$^{-1}$ (weak, O-H stretching).

$^1$H-NMR ($CD_3OD$): δ 7.10 (d, aromatic, 2H, $J_{P-H}$=2.6Hz), 5.01 (s, exchanged HOD), 2.99 (d, $-CH_2$, 2H, $J_{P-H}$ =21.2Hz), 1.41 (s, $-CH_3$, 18H).

$^{13}$C-NMR ($CD_3OD$): δ 152.9 (aromatic), 137.9 (aromatic), 126.2 (aromatic), 123.5 (aromatic), 34.41 (d, $-\underline{C}H_2$, 35.75, 33.07, $J_{P-C}$=537.2Hz), 34.35 ($-\underline{C}(CH_3)_3$), 29.7 ($-C(\underline{C}H_3)_3$).

$^{31}$P-NMR ($CD_3OD$): δ 26.8

The above-identified synthetic precursors included in the preparation of 3,5-Di-tert-butyl-4-hydroxybenzylphosphonic acid can be characterized by the following:

<u>Diethyl 3,5-di-tert-butyl-4-hydroxybenzylphosphonate</u>:

Melting point: 119-120°C (Lit: 118-119°C; Literature ref: R.K. Ismagilov, *Zhur. Obshchei Khimii,* **1991,** *61,* 387).

IR: 3451cm$^{-1}$ (weak, -OH, stretching), 2953 (weak, $-CH_3$, C-H stretching).

$^1$H-NMR ($CDCl_3$): δ 7.066 (d, Ar-H, 2H, $J_{P-H}$=2.8Hz), 5.145 (s, 1H, -OH), 4.06-3.92 (m, - $\underline{C}H_2CH_3$, 4H, H-H and long-range P-H couplings), 3.057 (d, Ar-$C\underline{H}_2$, 2H, $J_{P-H}$ =21.0 Hz), 1.412 (s, $-C(C\underline{H}_3)_3$, 18H), 1.222 (t, $-CH_2C\underline{H}_3$, 6H).

$^{13}$C-NMR ($CDCl_3$): δ 153.98 (aromatic), 136.22 (aromatic), 126.61 (aromatic), 122.07 (aromatic), 62.14 (-O$\underline{C}H_2CH_3$, $J_{P-C}$=24.4 Hz), 33.63 (Ar-$\underline{C}H_2$, $J_{P-C}$ = 552.4 Hz), 34.53 [-$\underline{C}(CH_3)_3$], 30.54 [-C($\underline{C}H_3)_3$], 16.66 (-$CH_2\underline{C}H_3$, $J_{P-C}$ =24.4 Hz).

$^{31}$P-NMR ($CDCl_3$): δ 28.43.

3,5-di-tert-butyl-4-hydroxybenzyl bromide:

Melting point: 51-54°C (Lit: 52-54°C; Literature ref: J.D. McClure, *J. Org. Chem.,* **1962**, *27*, 2365)

IR: 3616cm$^{-1}$ (medium, O-H stretching), 2954cm$^{-1}$ (weak, alkyl C-H stretching).

$^{1}$H-NMR ($CDCl_3$): δ 7.20 (s, Ar-H, 2H), 5.31 (s, -OH), 4.51 (s, -$CH_2$, 2H), 1.44 {s, [-C(C$\underline{H}_3)_3$], 18H}.

$^{13}$C-NMR ($CDCl_3$): δ 154.3 (aromatic), 136.5 (aromatic), 128.7 (aromatic), 126.3 (aromatic), 35.8 [(-$\underline{C}(CH_3)_3$], 34.6 (-$CH_2$), 30.5 [-C($\underline{C}H_3)_3$].

Other synthetic approaches that are known or readily ascertainable by one of ordinary skill in the relevant art can be used to prepare 3,5-Di-tert-butyl-4-hydroxybenzylphosphonic acid.

***Synthesis of CdSe/CdZnS Core-Shell Nanocrystals:***

25.86 mmol of trioctylphosphine oxide and 2.4 mmol of 3, 5-di-*tert*-butyl-4-hydroxybenzylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over the course of 2 hours at 155°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in chloroform and used to make semiconductor nanocrystal composite materials.

In Table 2, the 3,5-di-*tert*-butyl-4-hydroxybenzylphosphonic acid ligand group is referred to as BHT.

***Preparation of Layer including Semiconductor Nanocrystals:***

Films listed in Table 2 below are prepared using samples including semiconductor nanocrystals prepared substantially in accordance with the synthesis described in Example 5. Bulk chloroform is removed from the nanocrystal samples with nitrogen purging. Residual chloroform is removed from the semiconductor nanocrystals under vacuum at room temperature. Care is taken not to overdry or completely remove all solvent.

37 ml of RD-12, a low viscosity reactive diluent commercially available from Radcure Corp, 9 Audrey Pl, Fairfield. NJ 07004-3401, United States, is added to 4.68 gram of semiconductor nanocrystals under vacuum. The vessel is then backfilled with nitrogen and the mixture is mixed using a vortex mixer. After the semiconductor nanocrystals are pre-solubilized in the reactive diluent, 156 ml of DR-150, an UV-curable acrylic formulation commercially available Radcure,, is added slowly under vacuum. The vessel is then backfilled with nitrogen and the mixture is mixed using a vortex mixer.

2.00 gram TiO2 (if indicated) is next added and the mixture is mixed with an homogenizer.

12.00 gram curing agent Escacure TPO is added , following which the mixture is mixed with an homogenizer. The vessel including the mixture is then wrappered with black tape to shield the fluid from light.

The vessel in then backfilled with nitrogen and sonified for at least about 3 hours. Care is taken to avoid temperatures over 40C while the sample is in the ultrasonic bath.

Samples are coated by Mayer rod on precleaned glass slides and cured in a 5000-EC UV Light Curing Flood Lamp from DYMAX Corporation system with an H-bulb (225 mW/cm$^2$) for 10 seconds.

A sample is removed for evaluation and coated on a glass slide with a 52 rod and cured for 10 sec:

Thickness = 72 μm

(continued)

| | | | |
|---|---|---|---|
| Lambda em = | 633.1 nm | FWHM = 36 nm | |
| %EQE = | 50.0% | $\%A_{450\ nm}$ = 82.6% | |

Occasionally, the mixing vial is heated to lower viscosity and aid stirring. After the addition is competed, vacuum is pulled to remove entrained air. The vial is then placed in an ultrasonic bath (VWR) from 1 hour to overnight, resulting in a clear, colored solution. Care is taken to avoid temperatures over 40C while the sample is in the ultrasonic bath.

Multiple batches of the semiconductor nanocrystals of the same color are mixed together. Prior to making the acrylic preparation. Samples are coated by Mayer rod on precleaned glass slides and cured in a 5000-EC UV Light Curing Flood Lamp from DYMAX Corporation system with an H-bulb (225 $mW/cm^2$) for 10 seconds.

Samples including multiple layers for achieving the desired thickness are cured between layers. Samples including filters on top of (or below) the layers including host material and quantum confined semiconductor nanoparticles have the filters coated by Mayer rod in a separate step.

Filters are made by blending UV-curable pigment ink formulations from Coates/Sun Chemical. (Examples include, but are not limited to, DXT-1935 and WIN99.) A filter composition is formulated by adding the weighted absorbances of the individual colors together to achieve the desired transmission characteristics.

**Table 2**

| Film Color/Sample # (Nanocrystal Prep. Example #) | Solvent | Ligand(s) | Emission (nm) | FWHM | Film EQE (%) |
|---|---|---|---|---|---|
| Red/Sample #1 (without TiO2) (Ex. 5) | Chloroform | BHT | 631 | 36 | 29.0 |
| Red/Sample # 2 (with TiO2) (Ex. 5) | Chloroform | BHT | 633 | 36 | 50.0 |

**Film Characterization:**

The films are characterized in the following ways:

- Thickness: measured by a micrometer
- Emission measurement measured on sample 1 off each type, on Cary Eclipse. Excitation at 450nm, 2.5nm excitation slit, 5nm emission slit.
- Absorption measured at 450 nm on sample 1 of each type, on Cary 5000. Baseline corrected to blank glass slide.
- CIE coordinates measured on sample 1 of each type using CS-200 Chroma Meter. Sample excited with 450nm LED, and camera collected color data off axis.
- The external photoluminescent (PL) quantum efficiency is measured using the method developed by Mello et al. (1). The method uses a collimated 450 nm LED source, an integrating sphere and a spectrometer. Three measurements are taken. First, the LED directly illuminates the integrating sphere giving the spectrum labeled L1 in Figure 8. Next, the PL sample is placed into the integrating sphere so that only diffuse LED light illuminates the sample giving the (L2+P2) spectrum depicted in Figure 8. Finally, the PL sample is placed into the integrating sphere so that the LED directly illuminates the sample (just off normal incidence) giving the (L3+P3) spectrum depicted in Figure 8. After collecting the data, each spectral contribution (L's and P's) is computed. L1, L2 and L3 correspond to the sums of the LED spectra for each measurement and P2 and P3 are the sums associated with the PL spectra for 2nd and 3rd measurements. The following equation then gives the external PL quantum efficiency:

$$EQE = [(P3 \bullet L2) \text{ minus } (P2 \bullet L3)] / (L1 \bullet (L2 \text{ minus } L3))$$

For additional information concerning EQE measurements, see Mello et al., Advanced Materials 9(3):230(1997).

In certain embodiments, semiconductor nanocrystals are purified before deposition.

In certain embodiments, a desired ligand can be attached to a semiconductor nanocrystal by building the desired functionality into the phosphonic acid derivative, amine derivative, or both. Following is a non-limiting example of a schematic of a general synthetic procedure for generating a desired phosphonic acid derivative:

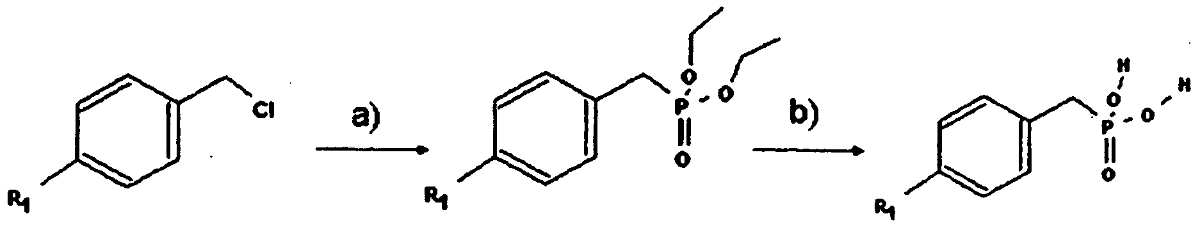


1 2 3

a) NaH, THF, NaI and 1.

b) 1. TMSBr, $CH_2Cl_2$. 2. $H_2O$.

Also refer to The Chemistry of Organophosphorus Compounds, Volume 4: Ter- and Quinque-Valent Phosphorus Acids and Their Derivatives, Frank R. Hartley (Editor), April 1996 for more general synthetic procedures for generating phosphonic acid derivatives.

In certain additional embodiments, a desired ligand can be attached to a semiconductor nanocrystal by building the desired functionality into the phosphonic acid derivative, amine derivative, or both. Following is a non-limiting example of a schematic of a general synthetic procedure for generating a desired amine derivative :

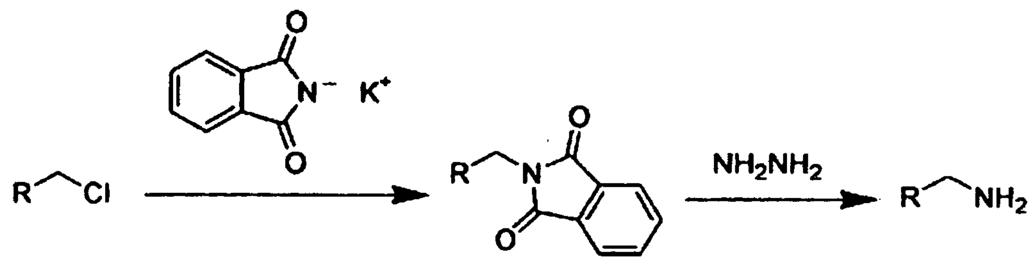


### Example 6 - Comparison of Semiconductor Nanocrystals Prepared With Native Ligands & Semiconductor Nanocrystals with Cap Exchanged Ligands

*Example 6A - Preparation of Semiconductor Nanocrystals Capable of Emitting Red Light Including Native Ligands*

*Synthesis of CdSe Cores:* 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

*Synthesis of CdSe/CdZnS Core-Shell Nanocrystals:* 25.86 mmol of trioctylphosphine oxide and 2.4 mmol of octadecylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure and then 2.4 mmol of 6-amino-1-hexanol was added to the reaction mixture. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over

the course of 2 hours at 155°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in hexane and their solution-state quantum yield assessed. (QY - 80%)

*Example 6B - Preparation of Cap Exchanged Semiconductor Nanocrystals Capable of remitting Red Light* (Reference)

*Synthesis of CdSe Cores:* 1 mmol cadmium acetate was dissolved in 8.96 mmol of tri-n-octylphosphine at 100°C in a 20 mL vial and then dried and degassed for one hour. 15.5 mmol of trioctylphosphine oxide and 2 mmol of octadecylphosphonic acid were added to a 3-neck flask and dried and degassed at 140°C for one hour. After degassing, the Cd solution was added to the oxide/acid flask and the mixture was heated to 270°C under nitrogen. Once the temperature reached 270°C, 8 mmol of tri-n-butylphosphine was injected into the flask. The temperature was brought back to 270°C where 1.1 mL of 1.5 M TBP-Se was then rapidly injected. The reaction mixture was heated at 270°C for 15-30 minutes while aliquots of the solution were removed periodically in order to monitor the growth of the nanocrystals. Once the first absorption peak of the nanocrystals reached 565-575 nm, the reaction was stopped by cooling the mixture to room temperature. The CdSe cores were precipitated out of the growth solution inside a nitrogen atmosphere glovebox by adding a 3:1 mixture of methanol and isopropanol. The isolated cores were then dissolved in hexane and used to make core-shell materials.

*Synthesis of CdSe/CdZnS Core-Shell Nanocrystals:* 25.86 mmol of trioctylphosphine oxide and 2.4 mmol of octadecylphosphonic acid were loaded into a four-neck flask. The mixture was then dried and degassed in the reaction vessel by heating to 120°C for about an hour. The flask was then cooled to 75°C and the hexane solution containing isolated CdSe cores (0.1 mmol Cd content) was added to the reaction mixture. The hexane was removed under reduced pressure and then 2.4 mmol of decylamine was added to the reaction mixture. Dimethyl cadmium, diethyl zinc, and hexamethyldisilathiane were used as the Cd, Zn, and S precursors, respectively. The Cd and Zn were mixed in equimolar ratios while the S was in two-fold excess relative to the Cd and Zn. The Cd/Zn and S samples were each dissolved in 4 mL of trioctylphosphine inside a nitrogen atmosphere glove box. Once the precursor solutions were prepared, the reaction flask was heated to 155°C under nitrogen. The precursor solutions were added dropwise over the course of 2 hours at 155°C using a syringe pump. After the shell growth, the nanocrystals were transferred to a nitrogen atmosphere glovebox and precipitated out of the growth solution by adding a 3:1 mixture of methanol and isopropanol. The isolated core-shell nanocrystals were then dissolved in hexane and used for cap exchange reactions. (QY ~ 80%)

*Cap-exchange reaction (1) on CdSe/CdZnS Core-Shell Nanocrystals:* 10 mL of toluene and 42.6 mmol of 6-amino-1-hexanol were loaded into a four-neck flask. The flask was evacuated and refilled with nitrogen three times. The flask was then heated to 40°C and the hexane solution containing isolated CdSe/CdZnS core/shell (1 prep) was added to the reaction mixture. The mixture was heated at 40°C overnight. Finally, the cap-exchanged semiconductor nanocrystals were precipitated out of the growth solution by adding hexane. The isolated core-shell nanocrystals were then dissolved in a 3:1 methanol and isopropanol mixture and their solution-state quantum yield assessed. (QY ~ 15%)

*Cap-exchange reaction* (2) *on CdSe/CdZnS Core-Shell Nanocrystals:* 25.86 mmol of trioctylphosphine oxide, 2.4 mmol of octadecylphosphonic acid, and 2.4 mmol of 6-amino-1-hexanol were loaded into a four-neck flask. The mixture was then dried and degassed in the reaction vessel by heating to 120°C for about an hour. The flask was then cooled to 40°C and the hexane solution containing isolated CdSe/CdZnS core/shell (1 prep) was added to the reaction mixture. The mixture was heated at 40°C overnight. Finally, the cap-exchanged semiconductor nanocrystals were precipitated out of the growth solution by adding a 3:1 mixture of methanol and isopropanol. The isolated core-shel nanocrystals were then dissolved in hexane and their solution-state quantum yield assessed. (QY ~ 19%)

Nanoparticles can have various shapes, including, but not limited to, sphere, rod, disk, other shapes, and mixtures of various shaped particles.

Metallic nanoparticles can be prepared as described, for example, in U.S. Patent No. 6,054,495. The metallic nanoparticle can be a noble metal nanoparticle, such as a gold nanoparticle. Gold nanoparticles can be prepared as described in U.S. Patent No. 6,506,564. Ceramic nanoparticles can be prepared as described, for example, in U.S. Patent No. 6,139,585.

Narrow size distribution, high quality semiconductor nanocrystals with high fluorescence efficiency can be prepared using previously established literature procedures and used as the building blocks. *See,* C.B. Murray et al., J. Amer. Chem. Soc. 1993, 115, 8706, B.O. Dabbousi et al., J. Phys. Chem. B 1997, 101, 9463 Other methods known or readily ascertainable by the skilled artisan can also be used.

In certain embodiments, nanoparticles comprise chemically synthesized colloidal nanoparticles (nanoparticles), such as semiconductor nanocrystals or quantum dots. In certain preferred embodiments, the nanoparticles (e.g., semiconductor nanocrystals) have a diameter in a range from about 1 to about 10 nm. In certain embodiments, at least a portion of the nanoparticles, and preferably all of the nanoparticles, include one or more ligands attached to a surface of a nanoparticle. *See,* C. B. Murray et al., Annu. Rev. Mat. Sci., 30, 545-610 (2000). These zero-dimensional structures

show strong quantum confinement effects that can be harnessed in designing bottom-up chemical approaches to create complex heterostructures with electronic and optical properties that are tunable with the size of the nanocrystals.

Emission from semiconductor nanocrystals can occur at an emission wavelength when one or more of the nanocrystals is excited. The emission has a frequency that corresponds to the band gap of the quantum confined semiconductor material. The band gap is a function of the size of the nanocrystal. Nanocrystals having small diameters can have properties intermediate between molecular and bulk forms of matter. For example, nanocrystals based on semiconductor materials having small diameters can exhibit quantum confinement of both the electron and hole in all three dimensions, which leads to an increase in the effective band gap of the material with decreasing crystallite size. Consequently, both the optical absorption and emission of nanocrystals shift to the blue (i.e., to higher energies) as the size of the crystallites decreases.

The emission from a nanocrystal can be a narrow Gaussian emission band that can be tuned through the complete wavelength range of the ultraviolet, visible, or infrared regions of the spectrum by varying the size of the nanocrystal, the composition of the nanocrystal, or both. The narrow size distribution of a population of nanocrystals can result in emission of light in a narrow spectral range. The population can be monodisperse and can exhibit less than a 15% rms deviation in diameter of the nanocrystals, preferably less than 10%, more preferably less than 5%. Spectral emissions in a narrow range of no greater than about 75 nm, preferably 60 nm, more preferably 40 nm, and most preferably 30 nm full width at half max (FWHM) can be observed. The breadth of the emission decreases as the dispersity of nanocrystal diameters decreases.

Semiconductor nanocrystals can have high emission quantum efficiencies such as greater than 10%, 20%, 30%, 40%, 50%, 60%, 70%, or 80%. The semiconductor forming the nanocrystals can include Group IV elements, Group II-VI compounds, Group II-V compounds, Group III-VI compounds, Group III-V compounds, Group IV-VI compounds, Group I-III-VI compounds, Group II-IV-VI compounds, or Group II-IV-V compounds, for example, ZnS, ZnSe, ZnTe, CdS, CdSe, CdTe, HgS, HgSe, HgTe, AlN, AlP, AlAs, AlSb, GaN, GaP, GaAs, GaSb, GaSe, InN, InP, InAs, InSb, TlN, TIP, TlAs, TlSb, PbS, PbSe, PbTe, or mixtures thereof.

Examples of methods of preparing monodisperse semiconductor nanocrystals include pyrolysis of organometallic reagents, such as dimethyl cadmium, injected into a hot, coordinating solvent. This permits discrete nucleation and results in the controlled growth of macroscopic quantities of nanocrystals. Preparation and manipulation of nanocrystals are described, for example, in U.S. Patent 6,322,901. Such methods of manufacturing nanocrystals involve a colloidal growth process. Colloidal growth occurs by rapidly injecting an M donor and an X donor into a hot coordinating solvent. The injection produces a nucleus that can be grown in a controlled manner to form a nanocrystal. The reaction mixture can be gently heated to grow and anneal the nanocrystal. Both the average size and the size distribution of the nanocrystals in a sample are dependent on the growth temperature. The growth temperature necessary to maintain steady growth increases with increasing average crystal size. The nanocrystal is a member of a population of nanocrystals. As a result of the discrete nucleation and controlled growth, the population of nanocrystals obtained has a narrow, monodisperse distribution of diameters. The monodisperse distribution of diameters can also be referred to as a size. The process of controlled growth and annealing of the nanocrystals in the coordinating solvent that follows nucleation can also result in uniform surface derivatization and regular core structures. As the size distribution sharpens, the temperature can be raised to maintain steady growth. By adding more M donor or X donor, the growth period can be shortened.

The M donor can be an inorganic compound, an organometallic compound, or elemental metal. For example, M can be cadmium, zinc, magnesium, mercury, aluminum, gallium, indium or thallium. The X donor is a compound capable of reacting with the M donor to form a material with the general formula MX. Typically, the X donor is a chalcogenide donor or a pnictide donor, such as a phosphine chalcogenide, a bis(silyl) chalcogenide, dioxygen, an ammonium salt, or a tris(silyl) pnictide. Suitable X donors include dioxygen, bis(trimethylsilyl) selenide ($(TMS)_2Se$), trialkyl phosphine selenides such as (tri-n-octylphosphine) selenide (TOPSe) or (tri-n-butylphosphine) selenide (TBPSe), trialkyl phosphine tellurides such as (tri-n-octylphosphine) telluride (TOPTe) or hexapropylphosphorustriamide telluride (HPPTTe), bis(trimethylsilyl)telluride ($(TMS)_2Te$), bis(trimethylsilyl)sulfide ($(TMS)_2S$), a trialkyl phosphine sulfide such as (tri-n-octylphosphine) sulfide (TOPS), an ammonium salt such as an ammonium halide (e.g., $NH_4Cl$), tris(trimethylsilyl) phosphide ($(TMS)_3P$), tris(trimethylsilyl) arsenide ($(TMS)_3As$), or tris(trimethylsilyl) antimonide ($(TMS)_3Sb$). In certain embodiments, the M donor and the X donor can be moieties within the same molecule.

A coordinating solvent can help control the growth of nanocrystals. The coordinating solvent is a compound having a donor lone pair that, for example, has a lone electron pair available to coordinate to a surface of the growing nanocrystal. Solvent coordination can stabilize the growing nanocrystal. Typical coordinating solvents include alkyl phosphines, alkyl phosphine oxides, alkyl phosphonic acids, or alkyl phosphinic acids, however, other coordinating solvents, such as pyridines, furans, and amines may also be suitable for the nanocrystal production. Examples of suitable coordinating solvents include pyridine, tri-n-octyl phosphine (TOP), tri-n-octyl phosphine oxide (TOPO) and tris-hydroxylpropylphosphine (tHPP). Technical grade TOPO can be used.

In certain methods, a non-coordinating or weakly coordinating solvent can be used.

Size distribution during the growth stage of the reaction can be estimated by monitoring the absorption line

widths of the particles. Modification of the reaction temperature in response to changes in the absorption spectrum of the particles allows the maintenance of a sharp particle size distribution during growth. Reactants can be added to the nucleation solution during crystal growth to grow larger crystals. By stopping growth at a particular nanocrystal average diameter and choosing the proper composition of the semiconducting material, the emission spectra of the nanocrystals can be tuned continuously over the wavelength range of 300 nm to 5 microns.

Semiconductor nanocrystals include, for example, inorganic crystallites between about 1 nm and about 1000 nm in diameter, preferably between about 2 nm and about 50 um, more preferably about 1 nm to about 20 nm (such as about 6, 7, 8, 9, 10, 11, 12, 13, 14, 15, 16, 17, 18, 19, or 20 nm).

A semiconductor nanocrystal typically has a diameter of less than 150 A. A population of nanocrystals preferably has average diameters in the range of 15 A to 125 A.

The nanocrystal can be a member of a population of nanocrystals having a narrow size distribution. The nanocrystal can be a sphere, rod, disk, or other shape. The nanocrystal can include a core of a semiconductor material. The nanocrystal can include a core having the formula MX, where M comprises one or more metals (e.g., but not limited to, cadmium, zinc, magnesium, mercury, aluminum, gallium, indium, thallium, or mixtures thereof), and X comprises one or more members of Group IV, V, or VI (e.g., but not limited to, oxygen, sulfur, selenium, tellurium, nitrogen, phosphorus, arsenic, antimony, or mixtures thereof). In certain embodiments, a nanocrystal can comprise a Group II-VI compound, Group II-V compound, Group III-VI compound, Group III-V compound, Group IV-VI compound, Group I-III-VI compound, Group II-IV-VI compound, and Group H-IV-V compound. In certain embodiments, a nanocrystal can comprise a Group IV element.

The core can have an overcoating on a surface of the core. The overcoating can be a semiconductor material having a composition different from the composition of the core. The overcoat of a semiconductor material on a surface of the nanocrystal can include a Group II-VI compound, Group II-V compound, Group III-VI compound, Group III-V compound, Group IV-VI compound, Group I-III-VI compound, Group II-IV-VI compound, and Group II-IV-V compound, for example, ZnS, ZnSe, ZnTe, CdS, CdSe, CdTe, HgS, HgSe, HgTe, AlN, AlP, AlAs, AlSb, GaN, GaP, GaAs, GaSb, GaSe, InN, InP, InAs, InSb, TlN, TIP, TlAs, TiSb, PbS, PbSe, PbTe, or mixtures thereof. In certain embodiments, a nanocrystal can comprise a Group IV element.

For example, ZnS, ZnSe or CdS overcoatings can be grown on CdSe or CdTe nanocrystals. An overcoating process is described, for example, in U.S. Patent 6,322,901. By adjusting the temperature of the reaction mixture during overcoating and monitoring the absorption spectrum of the core, over coated materials having high emission quantum efficiencies and narrow size distributions can be obtained.

The particle size distribution can be further refined by size selective precipitation with a poor solvent for the nanocrystals, such as methanol/butanol as described in U.S. Patent 6,322,901. For example, nanocrystals can be dispersed in a solution of 10% butanol in hexane. Methanol can be added dropwise to this stirring solution until opalescence persists. Separation of supernatant and flocculate by centrifugation produces a precipitate enriched with the largest crystallites in the sample. This procedure can be repeated until no further sharpening of the optical absorption spectrum is noted. Size-selective precipitation can be carried out in a variety of solvent/nonsolvent pairs, including pyridine/hexane and chloroform/methanol. The size-selected nanocrystal population can have no more than a 15% rms deviation from mean diameter, preferably 10% rms deviation or less, and more preferably 5% rms deviation or less.

Transmission electron microscopy (TEM) can provide information about the size, shape, and distribution of the nanocrystal population. Powder x-ray diffraction (XRD) patterns can provided the most complete information regarding the type and quality of the crystal structure of the nanocrystals. Estimates of size are also possible since particle diameter is inversely related, via the X-ray coherence length, to the peak width. For example, the diameter of the nanocrystal can be measured directly by transmission electron microscopy or estimated from x-ray diffraction data using, for example, the Scherrer equation. It also can be estimated from the UV/Vis absorption spectrum.

Narrow FWHM of nanocrystals can result in saturated color emission. This can lead to efficient nanocrystal-light emitting devices even in the red and blue parts of the spectrum, since in nanocrystal emitting devices no photons are lost to infrared and UV emission. The broadly tunable, saturated color emission over the entire visible spectrum of a single material system is unmatched by any class of organic chromophores. Furthermore, environmental stability of covalently bonded inorganic nanocrystals suggests that device lifetimes of hybrid organic/inorganic light emitting devices should match or exceed that of all-organic light emitting devices, when nanocrystals are used as luminescent centers. The degeneracy of the band edge energy levels of nanocrystals facilitates capture and radiative recombination of all possible excitons, whether generated by direct charge injection or energy transfer. The maximum theoretical nanocrystal-light emitting device efficiencies are therefore comparable to the unity efficiency of phosphorescent organic light emitting devices. The nanocrystal's excited state lifetime ($\tau$) is much shorter ($\tau \approx 10$ ns) than a typical phosphor ($\tau > 0.5$ $\mu$s), enabling nanocrystal-light emitting devices to operate efficiently even at high current density.

Semiconductor nanocrystals in accordance with the present inventions can be included in emissive materials for use in light-emitting devices, displays, and other optoelectronic and electronic devices, including, but not limited to, those described, for example, in International Application No. PCT/US2007/013152, entitled "Light-Emitting Devices

And Displays With Improved Performance", of QD Vision, Inc. *et al.,* filed 4 June 2007.

Semiconductor nanocrystals in accordance with the present inventions can be included in photoluminescent applications including, but not limited to, those described in U.S. Application No. 60/971885, of Coe-Sullivan, *et al.,* entitled "Optical Component, System Including An Optical Component, Devices, And Composition", filed 12 September 2007, and U.S. Application No. 60/973644, entitled "Optical Component, System Including An Optical Component, Devices, And Composition", of Coe-Sullivan, *et al.,* filed 19 September 2007.

Other materials, techniques, methods, applications, and information that may be useful with the present invention are described in International Patent Application No. PCT/US2007/ 24750, entitled "Improved Composites And Devices Including Nanoparticles", of Coe-Sullivan, *et al,* filed 3 December 2007, and U.S. Application No. 60/971887, entitled "Functionalized Semiconductor Nanocrystals And Method", of Breen, *et al.,* filed 12 September 2007; and International Application No. PCT/US2007/014711, entitled "Methods For Depositing Nanomaterial, Methods For Fabricating A Device, And Methods For Fabricating An Array Of Devices", of QD Vision, Inc. *et al.,* filed 25 June 2007.

As used herein, the singular forms "a", "an" and "the" include plural unless the context clearly dictates otherwise. Thus, for example, reference to an emissive material includes reference to one or more of such materials.

When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

The terms and expressions which have been employed herein are used as terms of description and not of limitation, and there is no intention in the use of such terms and expressions of excluding equivalents of the features shown and described, or portions thereof, it being recognized that various modifications are possible within the scope of the invention claimed. Moreover, any one or more features of any embodiment of the invention may be combined with any one or more other features of any other embodiment of the invention, without departing from the scope of the invention. Additional embodiments of the present invention will also be apparent to those skilled in the art from consideration of the specification and practice of the invention disclosed herein.

## Claims

**1.** A nanoparticle comprising a semiconductor nanocrystal including inorganic crystallites between 1 to 20 nm in diameter, the nanoparticle including one or more chemically distinct native ligands attached to a surface thereof, at least one of said ligands being represented by the formula:

X-Sp-Z

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle, and/or (ii) a group that is cyclic, halogenated, and/or polar aprotic, wherein Z in all cases is not reactive upon exposure to light, and wherein a native ligand is a ligand that attaches or coordinates to a nanoparticle surface during the growth or overcoating thereof with an overcoating material comprising a semiconductor material.

**2.** A method for functionalizing a nanoparticle comprising a semiconductor nanocrystal including inorganic crystallites between 1 to 20 nm in diameter, comprising reacting precursors for forming a nanoparticle having a predetermined composition in the presence of one or more chemically distinct ligands, at least one of the ligands being represented by the formula:

X-Sp-Z

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle and/or (ii) a group

that is cyclic, halogenated, and/or polar aprotic, wherein Z in all cases is not reactive upon exposure to light.

**3.** A method for overcoating at least a portion of a surface of a nanoparticle comprising a semiconductor nanocrystal including inorganic crystallites between 1 to 20 nm in diameter, with a coating material having a predetermined composition, the method comprising reacting precursors for the predetermined composition in the presence of one or more chemically distinct ligands, at least one of the ligands being represented by the formula:

X-Sp-Z

wherein X represents a primary amine group, a secondary amine group, a urea, a thiourea, an amide group, a phosphonic or arsonic acid group, a phosphinic or arsinic acid group, an arsenate group, a phosphine oxide or arsine oxide group; Sp represents a spacer group, such as a group capable of allowing a transfer of charge or an insulating group; and Z represents: (i) a reactive group capable of communicating specific chemical properties to the nanoparticle as well as provide specific chemical reactivity to the surface of the nanoparticle and/or (ii) a group that is cyclic, halogenated, and/or polar a-protic, wherein Z in all cases is not reactive upon exposure to light, and wherein the predetermined composition comprises a semiconductor material.

**4.** A nanoparticle in accordance with claim 1 or a method in accordance with claims 2 or 3 wherein Z does not render the nanoparticle dispersible in a liquid medium that includes water.

**5.** A nanoparticle in accordance with claim 1 or a method in accordance with claims 2 or 3 wherein the reactive group comprises a functional, bifunctional, or polyfunctional reagent, and/or a reactive chemical group.

**6.** A nanoparticle in accordance with claim 1 or a method in accordance with claims 2 or 3 wherein the cyclic group comprises a saturated or unsaturated cyclic or bicyclic compound or aromatic compound; or wherein the cyclic group includes at least one heteroatom and/or at least one substituent group.

**7.** A nanoparticle in accordance with claim 1 or a method in accordance with claims 2 or 3 wherein the halogenated group comprises a fluorinated group, perfluorinated group, a chlorinated group, a perchlorinated group, a brominated group, a perbrominated group, an iodinated group, a periodinated group.

**8.** A nanoparticle in accordance with claim 1 or a method in accordance with claims 2 or 3 wherein the polar aprotic group comprises a ketone, aldehyde, amide, urea, urethane, or an imine.

**9.** A nanoparticle in accordance with claim 1, wherein the nanoparticle includes two or more chemically distinct native ligands attached to a surface thereof, or a method in accordance with claim 2 or 3 wherein the precursors are reacted in the presence of two or more chemically distinct ligands, at least one of said ligands being represented by the formula:

X-Sp-Z.

**10.** A nanoparticle in accordance with claim 1 including two or more chemically distinct native ligands attached to a surface thereof, or a method in accordance with claim 2, 3 or 9 wherein the precursors are reacted in the presence of two or more chemically distinct ligands, wherein a first ligand is represented by the formula:

N-Sp-Z

wherein N represents a primary amine group, a secondary amine group, an amide group; and a second ligand is represented by the formula:

Y-Sp-Z

wherein Y represents a phosphonic or arsonic acid group, a phosphinic or arsinic acid group, an arsenate group, a phosphine or arsine oxide group; and wherein each of Sp and Z on the first ligand and on the second ligand can be the same or different.

**11.** A nanoparticle in accordance with claim 9 or 10 wherein the nanoparticle comprises a core comprising a first material and a shell disposed over at least a portion of a surface of the core, the shell comprising a second material; wherein preferably the first material comprises a semiconductor material; or wherein preferably the second material comprises

a semiconductor material; or wherein preferably one or more additional shells are disposed over at least a portion of a surface of the shell.

**12.** A nanoparticle in accordance with claim 1, wherein the overcoating forms a shell disposed over at least a portion of a surface of the nanoparticle.

**13.** A nanoparticle in accordance with claim 12, wherein one or more additional shells are disposed over at least a portion of a surface of the shell.

**14.** A nanoparticle in accordance with claim 1 or a method in accordance with claim 2 or 3 wherein the ligand represented by the formula X-Sp-Z comprises benzylphosphonic acid, benzylphosphonic acid including at least one substituent group on the ring of the benzyl group, a conjugate base of one of the foregoing acid, or a mixture including one or more of the foregoing; or wherein the ligand represented by the formula X-Sp-Z comprises 3, 5-di-*tert*-butyl-4-hydroxybenzylphosphonic acid, a conjugate base of the acid, or a mixture including one or more of the foregoing; or wherein the ligand represented by the formula X-Sp-Z comprises an organic amine including a terminal hydroxyl group or a fluorinated organic amine.

**15.** A method in accordance with claim 2 wherein the predetermined composition comprises one or more metals and one or more chalcogens or pnictogens

**16.** A method in accordance with claim 3, 10 or 15 wherein the precursors include one or more metal-containing precursors and one or more chalcogen-containing or pnictogen-containing precursors.

**17.** A method in accordance with claim 16 wherein the one or more metal-containing precursors and one or more chalcogen-containing precursors or pnictogen-containing precursors are reacted in the presence of two or more chemically distinct ligands, at least one of said ligands being represented by the formula:

X-Sp-Z.

**18.** A method in accordance with claim 2 or 3 wherein the reaction is carried out in a liquid medium.

**19.** A method in accordance with claim 18 wherein the liquid medium comprises a coordinating solvent; or a non-coordinating solvent.

**20.** A method in accordance with claim 16 or 18 wherein the mole ratio of total metal included in the one or more metal-containing precursors to total moles of ligand represented by the formula X-Sp-Z is in the range from about 1 : 0.1 to about 1 : 100; preferably in the range from about 1 : 1 to about 1 : 50; more preferably in the range from about 1 : 1 to about 1 : 30.

**21.** A method in accordance with claim 18 wherein the mole ratio of total moles of the liquid medium to total moles of ligand represented by the formula X-Sp-Z is in the range from about 500 : 1 to about 2 : 1; preferably in the range from about 100:1 to about 5 : 1; more preferably in the range from about 50 : 1 to about 5 : 1.

**22.** A method in accordance with claim 3 or 9 wherein one or more coating materials are formed over at least a portion of a surface of the nanoparticle; or a method in accordance with claim 9.

**23.** A method in accordance with claim 10 wherein the ligands represented by the formulae N-Sp-Z and Y-Sp-Z are initially present in an equimolar amount, the equimolar amount being determined based on the N group content of the first ligand and the acid group content of the second ligand.

**24.** A functionalized nanoparticle prepared in accordance with the method of claim 2.

**25.** A nanoparticle including an overcoating prepared in accordance with the method of claim 3.

**Patentansprüche**

**1.** Nanopartikel, umfassend einen Halbleiter-Nanokristall, einschließlich anorganischer Kristallite mit einem Durchmesser zwischen 1 und 20 nm, wobei das Nanopartikel einen oder mehrere chemisch verschiedene native Liganden beinhaltet, die auf einer Oberfläche desselben befestigt sind, wobei mindestens einer der Liganden dargestellt ist durch die Formel:

X-Sp-Z

wobei X eine primäre Amingruppe, eine sekundäre Amingruppe, einen Harnstoff, einen Thioharnstoff, eine Amidgruppe, eine Phosphon- oder Arsonsäuregruppe, eine Phosphin- oder Arsinsäuregruppe, eine Arsenatgruppe, eine Phosphinoxid- oder Arsinoxidgruppe darstellt; Sp eine Spacer-Gruppe, wie eine Gruppe, die einen Ladungstransfer ermöglichen kann, oder eine isolierende Gruppe ist; und Z darstellt: (i) eine reaktive Gruppe, die dem Nanopartikel bestimmte chemische Eigenschaften vermitteln kann sowie der Oberfläche des Nanopartikels eine spezifische chemische Reaktivität bereitstellen kann, und/oder (ii) eine Gruppe, die cyclisch, halogeniert, und/oder polar aprotisch ist, wobei Z in allen Fällen nicht auf Einwirkung von Licht reagiert, und wobei ein nativer Ligand ein Ligand ist, der an eine Nanopartikeloberfläche während des Wachstums oder des Beschichtens derselben mit einem Überzugsmaterial, das ein Halbleitermaterial umfasst, bindet oder koordiniert.

2. Verfahren zum Funktionalisieren eines Nanopartikels, umfassend einen Halbleiter-Nanokristall, einschließlich anorganischer Kristallite mit einem Durchmesser zwischen 1 und 20 nm, umfassend das Umsetzen von Vorstufen zur Bildung eines Nanopartikels mit einer vorbestimmten Zusammensetzung in Gegenwart von einem oder mehreren chemisch verschiedenen Liganden, wobei mindestens einer der Liganden dargestellt ist durch die Formel:

X-Sp-Z

wobei X eine primäre Amingruppe, eine sekundäre Amingruppe, einen Harnstoff, einen Thioharnstoff, eine Amidgruppe, eine Phosphon- oder Arsonsäuregruppe, eine Phosphin- oder Arsinsäuregruppe, eine Arsenatgruppe, eine Phosphinoxid- oder Arsinoxidgruppe darstellt; Sp eine Spacer-Gruppe, wie eine Gruppe, die einen Ladungstransfer ermöglichen kann, oder eine isolierende Gruppe ist; und Z darstellt: (i) eine reaktive Gruppe, die dem Nanopartikel bestimmte chemische Eigenschaften vermitteln kann sowie der Oberfläche des Nanopartikels eine spezifische chemische Reaktivität bereitstellen kann, und/oder (ii) eine Gruppe, die cyclisch, halogeniert, und/oder polar aprotisch ist, wobei Z in allen Fällen nicht auf eine Einwirkung von Licht reagiert.

3. Verfahren zum Beschichten mindestens eines Teils einer Oberfläche eines Nanopartikels, umfassend einen Halbleiter-Nanokristall, einschließlich anorganischer Kristallite mit einem Durchmesser zwischen 1 und 20 nm, mit einem Beschichtungsmaterial mit einer vorbestimmten Zusammensetzung, wobei das Verfahren das Umsetzen von Vorstufen für die vorbestimmte Zusammensetzung in Gegenwart von einem oder mehreren chemisch verschiedenen Liganden umfasst, wobei mindestens einer der Liganden dargestellt ist durch die Formel:

X-Sp-Z

wobei X eine primäre Amingruppe, eine sekundäre Amingruppe, einen Harnstoff, einen Thioharnstoff, eine Amidgruppe, eine Phosphon- oder Arsonsäuregruppe, eine Phosphin- oder Arsinsäuregruppe, eine Arsenatgruppe, eine Phosphinoxid- oder Arsinoxidgruppe darstellt; Sp eine Spacer-Gruppe, wie eine Gruppe, die einen Ladungstransfer ermöglichen kann, oder eine isolierende Gruppe ist; und Z darstellt: (i) eine reaktive Gruppe, die dem Nanopartikel bestimmte chemische Eigenschaften vermitteln kann sowie der Oberfläche des Nanopartikels eine spezifische chemische Reaktivität bereitstellen kann, und/oder (ii) eine Gruppe, die cyclisch, halogeniert, und/oder polar aprotisch ist, wobei Z in allen Fällen nicht auf eine Einwirkung von Licht reagiert, und wobei die vorbestimmte Zusammensetzung ein Halbleitermaterial umfasst.

4. Nanopartikel nach Anspruch 1 oder Verfahren nach den Ansprüchen 2 oder 3, wobei Z das Nanopartikel nicht in einem flüssigen Medium, das Wasser enthält, dispergierbar macht.

5. Nanopartikel nach Anspruch 1 oder Verfahren nach den Ansprüchen 2 oder 3, wobei die reaktive Gruppe ein funktionelles, bifunktionelles oder polyfunktionelles Reagens und/oder eine reaktive chemische Gruppe umfasst.

6. Nanopartikel nach Anspruch 1 oder Verfahren nach den Ansprüchen 2 oder 3, wobei die cyclische Gruppe eine gesättigte oder ungesättigte cyclische oder bicyclische Verbindung oder aromatische Verbindung umfasst; oder wobei die cyclische Gruppe mindestens ein Heteroatom und/oder mindestens eine Substituentengruppe beinhaltet.

**7.** Nanopartikel nach Anspruch 1 oder Verfahren nach den Ansprüchen 2 oder 3, wobei die halogenierte Gruppe eine fluorierte Gruppe, perfluorierte Gruppe, eine chlorierte Gruppe, eine perchlorierte Gruppe, eine bromierte Gruppe, eine perbromierte Gruppe, eine iodierte Gruppe, eine periodierte Gruppe umfasst.

**8.** Nanopartikel nach Anspruch 1 oder Verfahren nach den Ansprüchen 2 oder 3, wobei die polare aprotische Gruppe ein Keton, Aldehyd, Amid, Harnstoff, Urethan, oder ein Imin umfasst.

**9.** Nanopartikel nach Anspruch 1, wobei das Nanopartikel zwei oder mehr chemisch verschiedene native Liganden beinhaltet, die an einer Oberfläche desselben befestigt sind, oder Verfahren nach Anspruch 2 oder 3, wobei die Vorstufen in Gegenwart von zwei oder mehr chemisch verschiedenen Liganden umgesetzt werden, wobei mindestens einer der Liganden dargestellt ist durch die Formel:

X-Sp-Z.

**10.** Nanopartikel nach Anspruch 1, das zwei oder mehr chemisch verschiedene native Liganden beinhaltet, die auf einer Oberfläche desselben befestigt sind, oder Verfahren nach Anspruch 2, 3 oder 9, wobei die Vorstufen in Gegenwart von zwei oder mehr chemisch verschiedenen Liganden umgesetzt werden, wobei ein erster Ligand dargestellt ist durch die Formel:

N-Sp-Z

wobei N eine primäre Amingruppe, eine sekundäre Amingruppe, eine Amidgruppe darstellt; und ein zweiter Ligand dargestellt ist durch die Formel:

Y-Sp-Z

wobei Y eine Phosphon- oder Arsonsäuregruppe, eine Phosphin- oder Arsinsäuregruppe, eine Arsenatgruppe, eine Phosphin- oder Arsinoxidgruppe darstellt; und worin jedes aus Sp und Z an dem ersten Liganden und an dem zweiten Liganden gleich oder verschieden sein kann.

**11.** Nanopartikel nach Anspruch 9 oder 10, wobei das Nanopartikel einen Kern, der ein erstes Material umfasst, und eine Schale umfasst, die über mindestens einem Teil einer Oberfläche des Kerns angeordnet ist, wobei die Schale ein zweites Material umfasst; wobei vorzugsweise das erste Material ein Halbleitermaterial umfasst; oder wobei vorzugsweise das zweite Material ein Halbleitermaterial umfasst; oder wobei vorzugsweise eine oder mehrere zusätzliche Schalen über mindestens einem Teil einer Oberfläche der Schale angeordnet sind.

**12.** Nanopartikel nach Anspruch 1, wobei die Beschichtung eine Schale bildet, die über mindestens einem Teil einer Oberfläche des Nanopartikels angeordnet ist.

**13.** Nanopartikel nach Anspruch 12, wobei eine oder mehrere zusätzliche Schalen über mindestens einem Teil einer Oberfläche der Schale angeordnet sind.

**14.** Nanopartikel nach Anspruch 1 oder Verfahren nach Anspruch 2 oder 3, wobei der durch die Formel X-Sp-Z dargestellte Ligand Benzylphosphonsäure, Benzylphosphonsäure mit mindestens einer Substituentengruppe an dem Ring der Benzylgruppe, eine konjugierte Base einer der vorhergehenden Säuren, oder eine Mischung einer oder mehrerer der vorhergehenden umfasst; oder wobei der durch die Formel X-Sp-Z dargestellte Ligand 3,5-Di-*tert*-butyl-4-hydroxybenzylphosphonsäure, eine konjugierte Base der Säure oder einer Mischung einer oder mehrerer der vorhergehenden umfasst; oder wobei der durch die Formel X-Sp-Z dargestellte Ligand ein organisches Amin mit einer endständigen Hydroxylgruppe oder ein fluoriertes organisches Amin umfasst.

**15.** Verfahren nach Anspruch 2, wobei die vorbestimmte Zusammensetzung ein oder mehrere Metalle und ein oder mehrere Chalkogene oder Vertreter der Stickstoffgruppe (Pnictogene) umfasst.

**16.** Verfahren nach Anspruch 3, 10 oder 15, wobei die Vorstufen eine oder mehrere metallhaltige Vorstufen und ein oder mehrere Chalcogen-haltige oder Pnictogen-haltige Vorstufen beinhalten.

**17.** Verfahren nach Anspruch 16, wobei die eine oder mehreren metallhaltigen Vorstufen und eine oder mehreren Chalcogen-haltigen Vorstufen oder Pnictogen-haltigen Vorstufen in Gegenwart von zwei oder mehr chemisch verschiedenen Liganden umgesetzt werden, wobei mindestens einer der Liganden dargestellt ist durch die Formel:

X-Sp-Z.

**18.** Verfahren nach Anspruch 2 oder 3, wobei die Reaktion in einem flüssigen Medium durchgeführt wird.

**19.** Verfahren nach Anspruch 18, wobei das flüssige Medium ein koordinierendes Lösungsmittel oder ein nicht-koordinierendes Lösungsmittel umfasst.

**20.** Verfahren nach Anspruch 16 oder 18, wobei das Molverhältnis von Gesamtmetall, beinhaltet in der einen oder den mehreren metallhaltigen Vorstufe(n), zu den Gesamtmolen des durch die Formel X-Sp-Z dargestellten Liganden im Bereich von etwa 1:0,1 bis etwa 1:100; vorzugsweise im Bereich von etwa 1:1 bis etwa 1:50, stärker bevorzugt im Bereich von etwa 1:1 bis etwa 1:30 liegt.

**21.** Verfahren nach Anspruch 18, wobei das Molverhältnis von Gesamtmolen des flüssigen Mediums zu Gesamtmolen des durch die Formel X-Sp-Z dargestellten Liganden im Bereich von etwa 500:1 bis etwa 2:1; vorzugsweise im Bereich von etwa 100:1 bis etwa 5:1, stärker bevorzugt im Bereich von etwa 50:1 bis etwa 5:1 liegt.

**22.** Verfahren nach Anspruch 3 oder 9, wobei ein oder mehrere Beschichtungsmaterialien auf mindestens einem Teil einer Oberfläche des Nanopartikels gebildet werden; oder ein Verfahren nach Anspruch 9.

**23.** Verfahren nach Anspruch 10, wobei die durch die Formeln N-Sp-Z und Y-Sp-Z dargestellten Liganden zunächst in einer äquimolaren Menge vorhanden sind, wobei die äquimolare Menge bezogen auf den Gehalt an N-Gruppe des ersten Liganden und den Gehalt an Säuregruppe des zweiten Liganden bestimmt wird.

**24.** Funktionalisiertes Nanopartikel, hergestellt nach dem Verfahren von Anspruch 2.

**25.** Nanopartikel mit einer Beschichtung, hergestellt nach dem Verfahren von Anspruch 3.

## Revendications

**1.** Nanoparticule comprenant un nanocristal semi-conducteur comportant des cristallites inorganiques dont le diamètre est compris entre 1 nm et 20 nm, la nanoparticule comportant un ou plusieurs ligands natifs chimiquement différents attachés à une surface de celle-ci, au moins un desdits ligands étant représenté par la formule :

X-Sp-Z

dans laquelle X représente un groupe amine primaire, un groupe amine secondaire, une urée, une thio-urée, un groupe amide, un groupe acide phosphonique ou arsonique, un groupe acide phosphinique ou arsinique, un groupe arsénate, ou un groupe d'oxyde de phosphine ou d'arsine ; Sp représente un groupe d'espacement, tel qu'un groupe capable de permettre un transfert de charge ou un groupe d'isolation ; et Z représente : (i) un groupe réactif capable de conférer des propriétés chimiques spécifiques à la nanoparticule et de procurer une réactivité chimique spécifique à la surface de la nanoparticule, et/ou (ii) un groupe cyclique, halogéné et/ou polaire aprotique, dans laquelle Z n'est dans tous les cas pas réactif lors d'une exposition à la lumière, et dans laquelle un ligand natif est un ligand qui s'attache ou se coordonne à une surface de nanoparticule durant la croissance ou le revêtement de celle-ci avec un matériau de revêtement comprenant un matériau semi-conducteur.

**2.** Procédé de fonctionnalisation d'une nanoparticule comportant un nanocristal semi-conducteur comprenant des cristallites inorganiques dont le diamètre est compris entre 1 nm et 20 nm, comprenant la mise en réaction des précurseurs pour former une nanoparticule ayant une composition prédéterminée en présence d'un ou plusieurs ligands chimiquement différents, au moins un desdits ligands étant représenté par la formule :

X-Sp-Z

dans laquelle X représente un groupe amine primaire, un groupe amine secondaire, une urée, une thio-urée, un groupe amide, un groupe acide phosphonique ou arsonique, un groupe acide phosphinique ou arsinique, un groupe arsénate, ou un groupe d'oxyde de phosphine ou d'arsine ; Sp représente un groupe d'espacement, tel qu'un groupe

capable de permettre un transfert de charge ou un groupe d'isolation ; et Z représente : (i) un groupe réactif capable de conférer des propriétés chimiques spécifiques à la nanoparticule et de procurer une réactivité chimique spécifique à la surface de la nanoparticule, et/ou (ii) un groupe cyclique, halogéné et/ou polaire aprotique, dans laquelle Z n'est dans tous les cas pas réactif lors d'une exposition à la lumière.

**3.** Procédé de revêtement d'au moins une partie de la surface d'une nanoparticule comprenant un nanocristal semi-conducteur comportant des cristallites inorganiques dont le diamètre est compris entre 1 nm et 20 nm, avec un matériau de revêtement ayant une composition prédéterminée, le procédé comprenant la mise en réaction des précurseurs pour la composition prédéterminée en présence d'un ou plusieurs ligands chimiquement différents, au moins un desdits ligands étant représenté par la formule :

X-Sp-Z

dans laquelle X représente un groupe amine primaire, un groupe amine secondaire, une urée, une thio-urée, un groupe amide, un groupe acide phosphonique ou arsonique, un groupe acide phosphinique ou arsinique, un groupe arsénate, ou un groupe d'oxyde de phosphine ou d'arsine ; Sp représente un groupe d'espacement, tel qu'un groupe capable de permettre un transfert de charge ou un groupe d'isolation ; et Z représente : (i) un groupe réactif capable de conférer des propriétés chimiques spécifiques à la nanoparticule et de procurer une réactivité chimique spécifique à la surface de la nanoparticule, et/ou (ii) un groupe cyclique, halogéné et/ou polaire aprotique, dans laquelle Z n'est dans tous les cas pas réactif lors d'une exposition à la lumière, et dans lequel la composition prédéterminée comprend un matériau semi-conducteur.

**4.** Nanoparticule selon la revendication 1 ou procédé selon la revendication 2 ou 3, dans lequel Z ne rend pas la nanoparticule dispersible dans un milieu liquide qui comprend de l'eau.

**5.** Nanoparticule selon la revendication 1 ou procédé selon la revendication 2 ou 3, dans lequel le groupe réactif comprend un réactif fonctionnel, bifonctionnel ou polyfonctionnel, et/ou un groupe chimique réactif.

**6.** Nanoparticule selon la revendication 1 ou procédé selon la revendication 2 ou 3, dans lequel le groupe cyclique comprend un composé aromatique ou un composé cyclique ou bicyclique saturé ou insaturé ; ou dans lequel le groupe cyclique comprend au moins un hétéroatome et/ou au moins un groupe de substitution.

**7.** Nanoparticule selon la revendication 1 ou procédé selon la revendication 2 ou 3, dans lequel le groupe halogéné comprend un groupe fluoré, un groupe perfluoré, un groupe chloré, un groupe perchloré, un groupe bromé, un groupe perbromé, un groupe iodé ou un groupe periodé.

**8.** Nanoparticule selon la revendication 1 ou procédé selon la revendication 2 ou 3, dans lequel le groupe polaire aprotique comprend une cétone, un aldéhyde, une amide, une urée, un uréthane ou une imine.

**9.** Nanoparticule selon la revendication 1, dans laquelle la nanoparticule comprend deux ou plusieurs ligands natifs chimiquement différents attachés à une surface de celle-ci, ou procédé selon la revendication 2 ou 3, dans lequel les précurseurs réagissent en présence de deux ou plusieurs ligands chimiquement différents, au moins un desdits ligands étant représenté par la formule :

X-Sp-Z.

**10.** Nanoparticule selon la revendication 1 comportant deux ou plusieurs ligands natifs chimiquement différents attachés à une surface de celle-ci, ou procédé selon la revendication 2, 3 ou 9 dans lequel les précurseurs réagissent en présence de deux ou plusieurs ligands chimiquement différents, dans lequel un premier ligand est représenté par la formule :

N-Sp-Z

dans laquelle N représente un groupe amine primaire, un groupe amine secondaire ou un groupe amide ; et un deuxième ligand est représenté par la formule :

Y-Sp-Z

dans laquelle Y représente un groupe acide phosphonique ou arsonique, un groupe acide phosphinique ou arsinique, un groupe arsénate, ou un groupe d'oxyde de phosphine ou d'arsine ; et dans laquelle chacun des groupes Sp et Z sur le premier ligand et sur le deuxième ligand peut être identique ou différent.

**11.** Nanoparticule selon la revendication 9 ou 10, dans laquelle la nanoparticule comprend un noyau comportant un premier matériau et une enveloppe agencée sur au moins une partie d'une surface du noyau, l'enveloppe comprenant un deuxième matériau ; dans laquelle le premier matériau comprend de préférence un matériau semi-conducteur ; ou dans laquelle le deuxième matériau comprend de préférence un matériau semi-conducteur ; ou dans laquelle une ou plusieurs enveloppes additionnelles sont de préférence agencées sur au moins une partie d'une surface de l'enveloppe.

**12.** Nanoparticule selon la revendication 1, dans laquelle le revêtement forme une enveloppe agencée sur au moins une partie d'une surface de la nanoparticule.

**13.** Nanoparticule selon la revendication 12, dans laquelle une ou plusieurs enveloppes additionnelles sont agencées sur au moins une partie d'une surface de l'enveloppe.

**14.** Nanoparticule selon la revendication 1 ou procédé selon la revendication 2 ou 3, dans lequel le ligand représenté par la formule X-Sp-Z comprend de l'acide benzylphosphonique, de l'acide benzylphosphonique comportant au moins un groupe de substitution sur l'anneau du groupe benzyle, une base conjuguée d'un des acides qui précèdent, ou un mélange comportant un ou plusieurs de ceux qui précèdent ; ou dans lequel le ligand représenté par la formule X-Sp-Z comprend de l'acide 3, 5-di-tert-butyl-4-hydroxybenzylphosphonique, une base conjuguée de l'acide, ou un mélange comportant un ou plusieurs de ceux qui précèdent ; ou dans lequel le ligand représenté par la formule X-Sp-Z comprend une amine organique comportant un groupe hydroxyle terminal ou une amine organique fluorée.

**15.** Procédé selon la revendication 2, dans lequel la composition prédéterminée comprend un ou plusieurs métaux et un ou plusieurs chalcogènes ou pnictogènes.

**16.** Procédé selon la revendication 3, 10 ou 15 dans lequel les précurseurs comprennent un ou plusieurs précurseurs contenant un métal et un ou plusieurs précurseurs contenant un chalcogène ou un pnictogène.

**17.** Procédé selon la revendication 16, dans lequel lesdits un ou plusieurs précurseurs contenant un métal et lesdits un ou plusieurs précurseurs contenant un chalcogène ou un pnictogène sont mis en réaction en présence de deux ou plusieurs ligands chimiquement différents, au moins un desdits ligands étant représenté par la formule :

X-Sp-Z.

**18.** Procédé selon la revendication 2 ou 3, dans lequel la réaction est mise en oeuvre dans un milieu liquide.

**19.** Procédé selon la revendication 18, dans lequel le milieu liquide comprend un solvant de coordination ; ou un solvant de non-coordination.

**20.** Procédé selon la revendication 16 ou 18, dans lequel le rapport molaire du nombre total de moles de métal compris dans lesdits un ou plusieurs précurseurs contenant un métal par le nombre total de moles de ligand représenté par la formule X-Sp-Z est compris dans une plage d'environ 1:0,1 à 1:100 ; de préférence dans une plage d'environ 1:1 à 1:50 ; et de préférence encore dans une plage d'environ 1:1 à 1:30.

**21.** Procédé selon la revendication 18, dans lequel le rapport molaire du nombre total de moles de milieu liquide par le nombre total de moles de ligand représenté par la formule X-Sp-Z est compris dans une plage d'environ 500:1 à 2:1 ; de préférence dans une plage d'environ 100:1 à 5:1 ; et de préférence encore dans une plage d'environ 50:1 à 5:1.

**22.** Procédé selon la revendication 3 ou 9, dans lequel un ou plusieurs matériaux de revêtement sont formés par-dessus au moins une partie d'une surface de la nanoparticule ; ou procédé selon la revendication 9.

**23.** Procédé selon la revendication 10, dans lequel les ligands représentés par les formules N-Sp-Z et Y-Sp-Z sont initialement présents en quantité équimolaire, la quantité équimolaire étant déterminée sur base de la teneur en groupes N du premier ligand et de la teneur en groupes acides du second ligand.

**24.** Nanoparticule fonctionnalisée préparée selon le procédé de la revendication 2.

**25.** Nanoparticule comportant un revêtement préparée selon le procédé de la revendication 3.

**Figure 1**

| | Phosphonic Acid | Amine |
|---|---|---|
| Aliphatic | 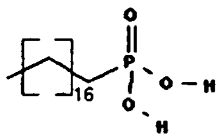 | 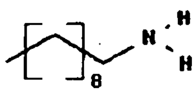 |
| Aromatic | 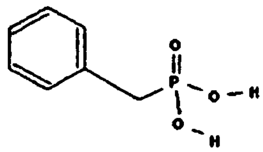 | 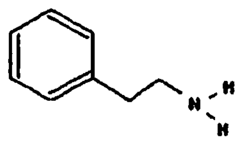 |

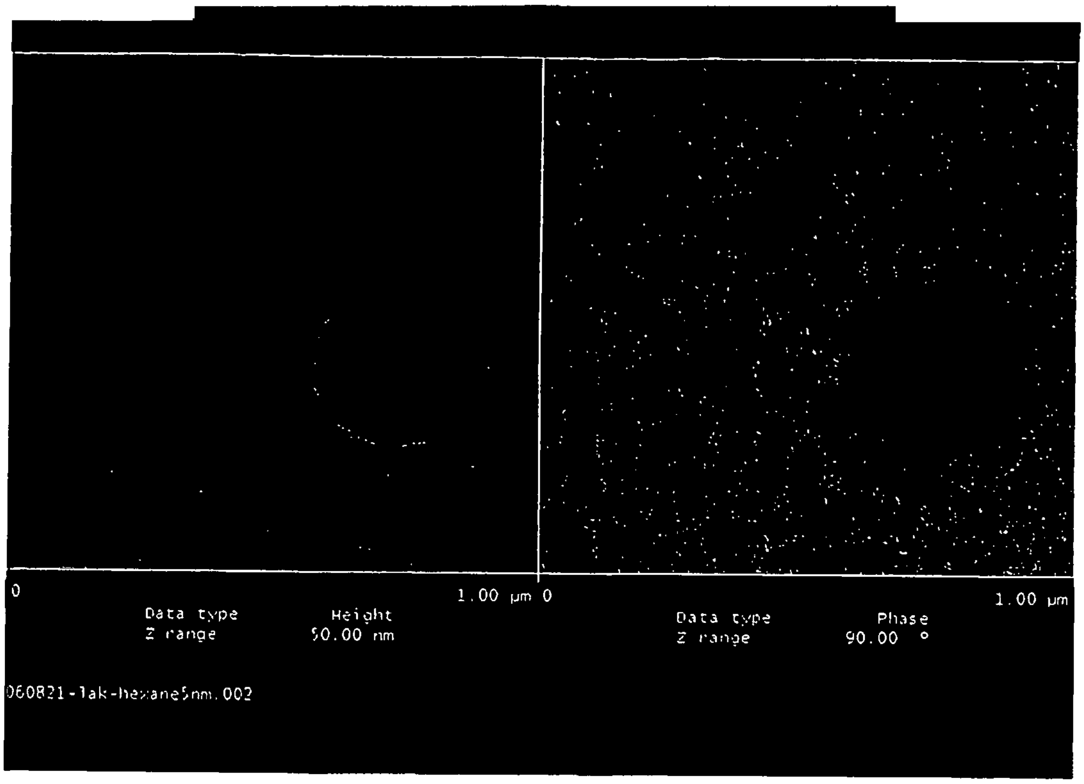


**Figure 2:** 5 nm CBP thermally evaporated on an aliphatic ligand quantum dot monolayer

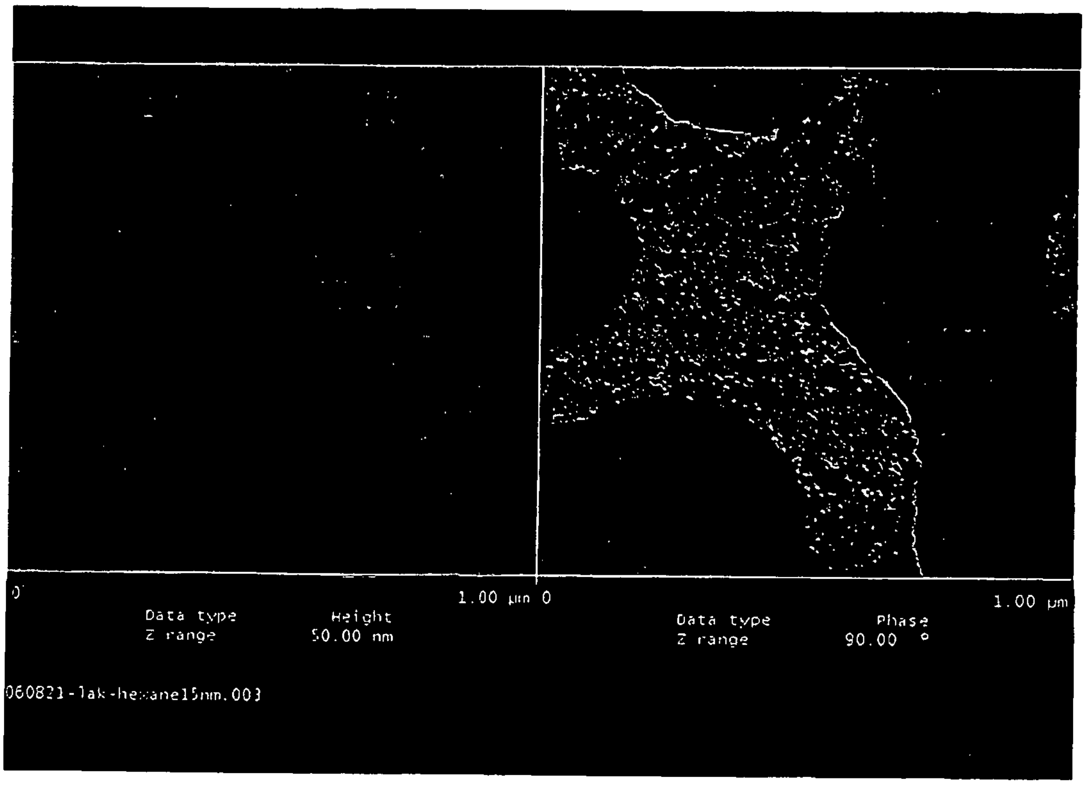


**Figure 3:** 15 nm CBP thermally evaporated on an aliphatic ligand quantum dot monolayer

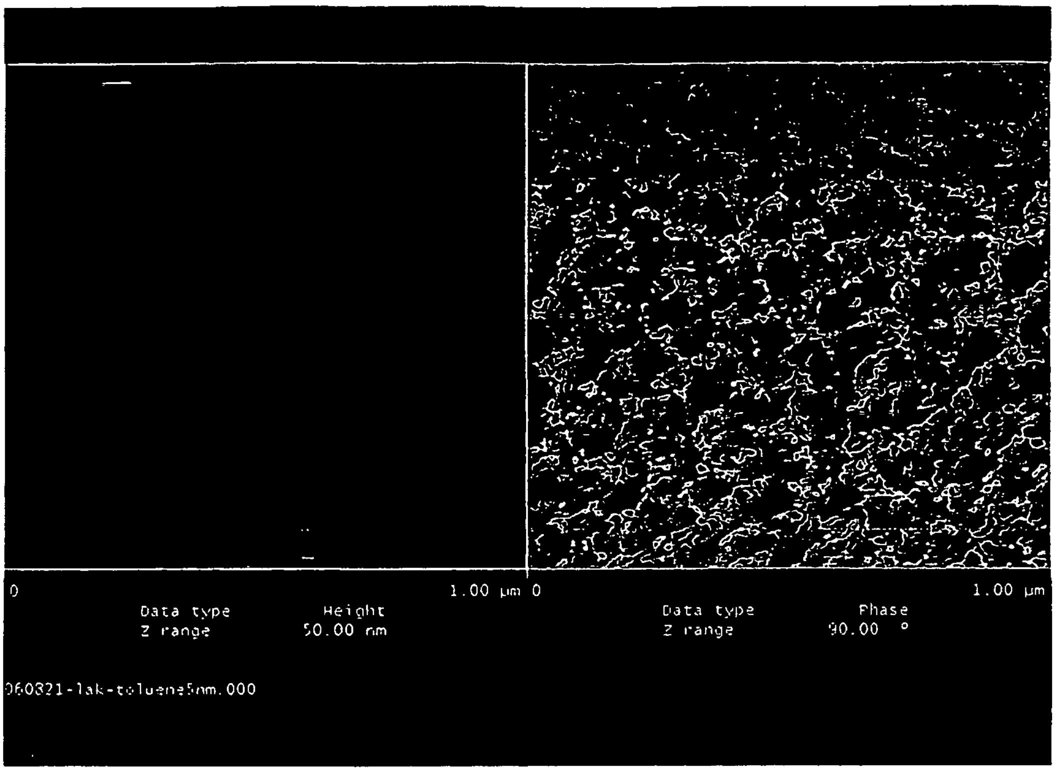


**Figure 4:** 5 nm CBP thermally evaporated on an aromatic ligand quantum dot monolayer

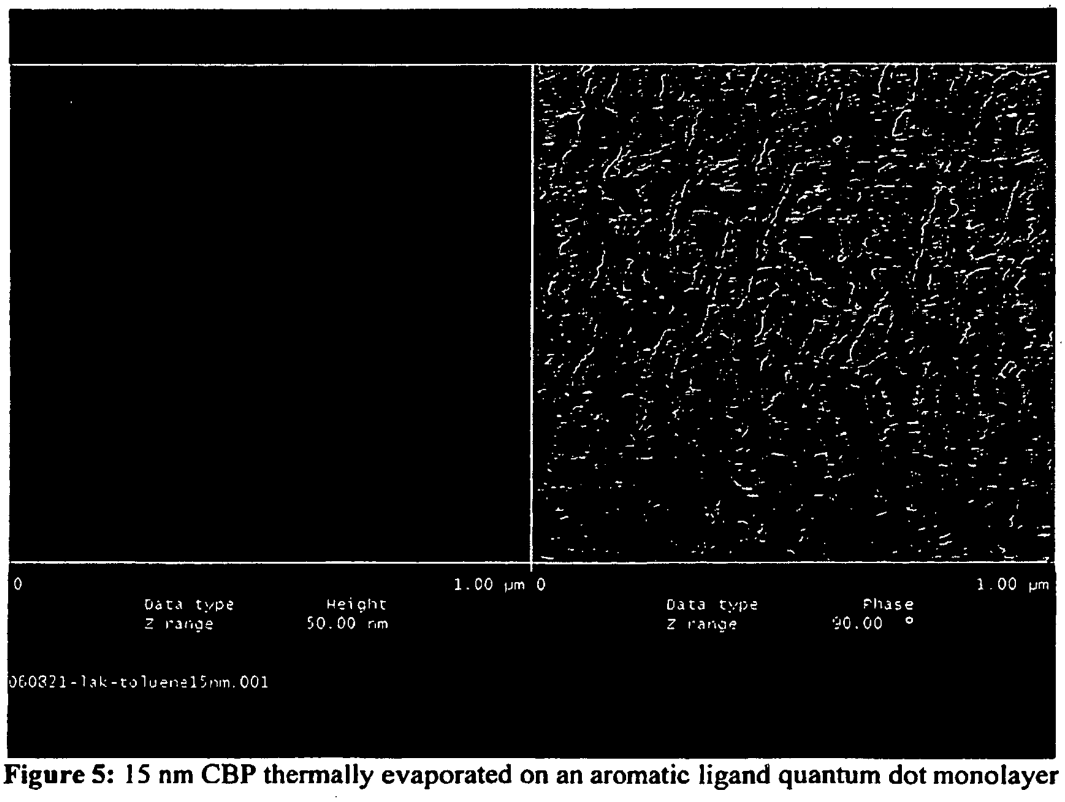


**Figure 5:** 15 nm CBP thermally evaporated on an aromatic ligand quantum dot monolayer

**Figure 6**

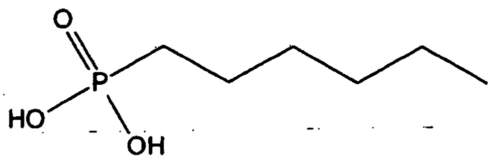

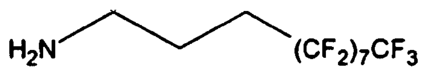

**Figure 7**

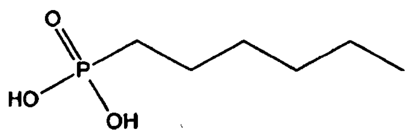

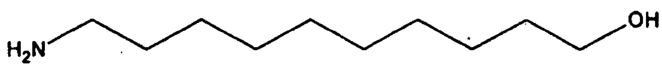

**Figure 8**

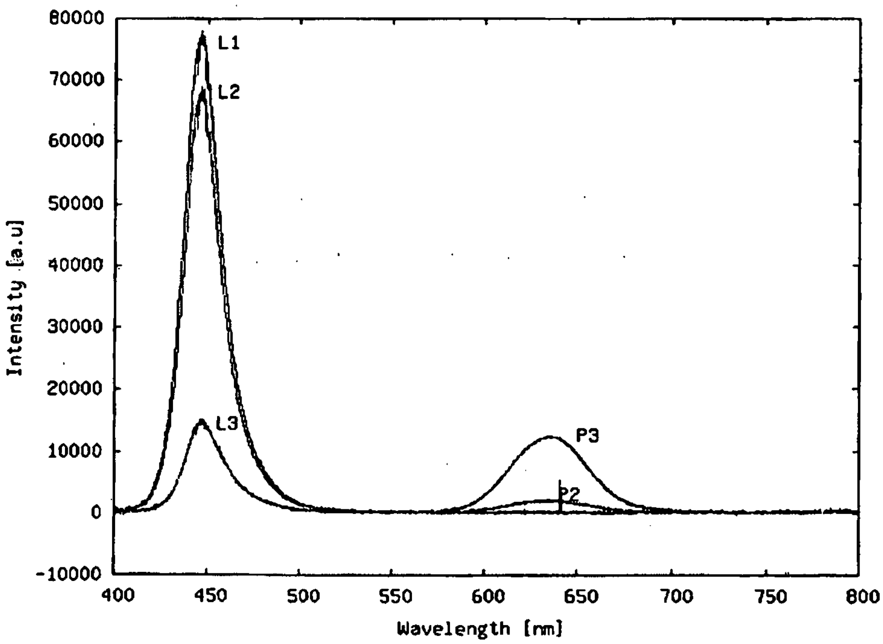

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060216759 A
- US 20030059635 A
- US 20050266246 A
- US 6054495 A
- US 6506564 B
- US 6139585 A
- US 6322901 B
- US 2007013152 W
- US 97188507 P
- US 97364407 P
- US 200724750 W
- US 97188707 P
- US 2007014711 W


### Non-patent literature cited in the description


- **DOUSSINEAU et al.** *Synlett,* 2004, vol. 10, 1735-1738
- **DIAMENTE et al.** *Langmuir,* 2006, vol. 22, 1782-1788
- **C.B. MURRAY et al.** *J. Amer. Chem. Soc.,* 1993, vol. 115, 8706
- **B.O. DABBOUSI et al.** *J. Phys. Chem. B,* 1997, vol. 101, 9463
- **C. B. MURRAY et al.** *Annu. Rev. Mat. Sci.,* 2000, vol. 30, 545-610